# EUROPÄISCHE PATENTSCHRIFT

(11) **EP 0 754 254 B1**
(45) Veröffentlichungstag und Bekanntmachung des Hinweises auf die Patenterteilung: **11.12.2002**
(21) Anmeldenummer: 96900820.0
(22) Anmeldetag: 30.01.1996
(51) Int. Cl.: D02G 1/12, G01N 33/36

(54) **VERFAHREN UND VORRICHTUNG ZUM KONTINUIERLICHEN KRÄUSELN VON THERMOPLASTISCHEN FÄDEN**
THERMOPLASTIC THREADS CONTINUOUS CRIMPING PROCESS AND DEVICE
PROCEDE ET DISPOSITIF POUR FRISER EN CONTINU DES FILS THERMOPLASTIQUES

(30) Priorität: 02.02.1995 CH 28495; 29.11.1995 CH 339495
(43) Veröffentlichungstag der Anmeldung: 22.01.1997
(73) Patentinhaber: MASCHINENFABRIK RIETER AG, 8406 Winterthur (CH)
(72) Erfinder: WEDER, Eugen, CH-8320 Fehraltorf (CH)
(86) Internationale Anmeldenummer: CH9600037
(87) Internationale Veröffentlichungsnummer: WO96023916

(56) Entgegenhaltungen:
- EP-A- 0 428 045
- EP-A- 0 554 642
- WO-A-93/16218
- DE-A- 2 933 782
- DE-A- 4 435 923
- DE-C- 3 609 216

## Beschreibung

Die Erfindung liegt auf dem Gebiete der Textiltechnik und betrifft ein Verfahren und eine Vorrichtung zum kontinuierlichen Kräuseln von Fäden aus einem thermoplastischen Material.

Zum kontinuierlichen Kräuseln von Fäden (Fibrillenbündel) aus thermoplastischem Material werden diese beispielsweise mit Hilfe eines unter Druck strömenden, heissen Fördermediums durch einen Förderkanal bewegt, dabei erhitzt und dann durch eine Austrittsöffnung in eine Stauchkammer gefördert, wobei die Stauchkammer derart gestaltet ist, dass sich das Fördermedium beim Austritt aus der Düsenaustrittsöffnung entspannt. Der Faden prallt in der Stauchkammer auf einen durch bereits aus der Öffnung ausgetretenen Faden gebildeten Pfropfen, wobei er gekräuselt wird. Der Pfropfen wird weitergefördert mit einer Geschwindigkeit, die kleiner ist als die Fadengeschwindigkeit im Förderkanal, wird dann abgekühlt und weiter zu einem texturierten Garn aufgelöst.

Die Stauchkammer, in der sich der Pfropfen bildet, kann begrenzt sein durch stationäre, den Pfropfen längsseitig umgebende, durchbrochene Wandungen, die beispielsweise aus Lamellen bestehen. Der Pfropfen wird durch den Staudruck des Fördermittels gegen die Reibung an den Stauchkammerwandungen durch die Stauchkammer gestossen und verlässt diese durch eine der Austrittsöffnung gegenüberliegende Pfropfenöffnung, die mit einem den Pfropfen dosiert austragenden Förderwalzenpaar versehen sein kann.

Die Stauchkammer kann auch nur teilweise von stationären Wandungen begrenzt sein und teilweise von Wandungen, die sich mit Pfropfengeschwindigkeit bewegen. Ein Verfahren und eine Vorrichtung zum kontinuierlichen Kräuseln von thermoplastischen Fäden mit einer Stauchkammer, die teilweise mit dem Pfropfen bewegende Wände aufweist, ist beispielsweise beschrieben in der europäischen Patentschrift Nr. 310890 derselben Anmelderin. Die beschriebene Vorrichtung weist eine Texturierdüse auf mit einem Förderkanal, einer Austrittsöffnung und zwei sich von dieser in Fadenlaufrichtung erstreckenden Anformungen, die die stationären Wandungsteile der Stauchkammer bilden. Zum Weitertransport des zwischen den Anformungen gebildeten Pfropfens dient ein durch seitliche Führungsmittel gebildeter, um den Umfang einer rotierenden Pfropfenförderwalze verlaufender Kanal, in den die Anformungen reichen. Die seitlichen Führungsmittel dieses Kanales stellen im Bereiche der Austrittsöffnung die mit dem Pfropfen bewegenden Teile der Stauchkammerwandungen dar. Der Pfropfen läuft von der Stauchkammer zwischen den seitlichen Führungsmitteln um einen Teil des Umfanges der Pfropfenförderwalze und wird dann auf ein weiteres Förderelement weitergegeben, wo er gekühlt und schliesslich zu einem texturierten Garn aufgelöst wird.

Der Faden wird mittels Fördermedium durch den Förderkanal und durch die Austrittsöffnung in die Stauchkammer gefördert. Unmittelbar ausserhalb der Austrittsöffnung entspannt sich das Fördermedium. Der Faden prallt auf den sich in der Stauchkammer bildenden Pfropfen auf und wird dabei gekräuselt. Der Pfropfen in der Stauchkammer wird nach dem Einführen des Fadens bei Produktionsbeginn durch eine temporäre Bremskraft, beispielsweise einen gegen den Faden gerichteten Luftstrahl initiiert. Während dem Betrieb besteht ein Gleichgewicht zwischen der den Pfropfen stossenden Staudruckkraft des Fördermediums und den den Pfropfen bremsenden Reibungskräften an den Wandungen der Stauchkammer, das zu einer kontinuierlichen Pfropfenbildung und einer konstanten Pfropfengeschwindigkeit in der Stauchkammer führt.

Die Qualität des texturierten Garnes hängt eng mit der Gleichmässigkeit der Kräuselung, das heisst mit der Gleichmässigkeit der Pfropfenbildung zusammen. Fehlt der Pfropfen, wird das Garn nicht gekräuselt. Beginnt die Pfropfenbildung zu weit von der Austrittsöffnung entfernt, wird der Pfropfen weniger dicht, so dass die Kräuselung nicht mehr genügend stark und genügend permanent wird. Dies bedeutet, dass für eine hohe Gamqualität Lage, Konsistenz und Geschwindigkeit des Pfropfens möglichst konstant gehalten werden müssen.

In allen bekannten Vorrichtungen zur Kräuselung von Fäden aus thermoplastischem Material mit Hilfe einer Texturierdüse und einer Stauchkammer können aber Unregelmässigkeiten in der Pfropfenbildung entstehen, vor allem Zustände, in denen die Pfropfenbildung sich zu weit von der Austrittsöffnung entfernt oder sich gar kein Pfropfen bildet, sogenannte Ausblaser. Je nach Vorrichtung sind diese Ausblaser temporär, das heisst, sie bilden sich ohne Einflussnahme zurück oder sie sind stationär, das heisst, die Maschine muss gestoppt werden, um wieder eine reguläre Pfropfenbildung zu erhalten.

Wenn Fehler in der Pfropfenbildung nur visuell entdeckt werden können, werden sie oft nicht oder spät entdeckt, so dass Spulen des texturierten Garnes Fehlerstellen aufweisen, die von unentdeckten, temporären Ausblasern herrühren und die erst an einem aus dem Garn hergestellten Produkt erkannt werden. Durch stationäre Ausblaser, die erst nach einer Weile entdeckt werden, können grosse Mengen von Ausschussgarn produziert werden.

Es war die Aufgabe der Erfindung, nach der schweizerischen Patentanmeldung 2052/92 vom 30. Juni 1992, ein Verfahren und eine Vorrichtung zum kontinuierlichen Kräuseln von Fäden aus thermoplastischem Material aufzuzeigen, mit denen Einbussen in der Garnqualität und Produktion von Ausschuss bedingt durch eine unstabile Pfropfenbildung, insbesondere bedingt durch Ausblaser, vermieden werden können. Diese Aufgabe wird gelöst durch ein Verfahren, wonach zum kontinuierlichen Kräuseln eines Fadens aus einem thermoplastischen Material, der Faden erwärmt, mit Hilfe eines strömenden Fördermediums mit einer Fadengeschwindigkeit durch einen Förderkanal und durch eine Austrittsöffnung in eine Stauchkammer gefördert, durch Bremsung in der Stauchkammer zu einem Pfropfen gestaucht und in dieser Form mit einer Pfropfengeschwindigkeit, die kleiner ist als die Fadengeschwindigkeit, weitergefördert wird zur Kühlung und Auflösung, wobei im Bereiche der Austrittsöffnung die Pfropfenbildung sensorisch überwacht wird, und die durch die Überwachung erzeugten Messsignale als Messgrössen für einen geschlossenen Regelkreis zur Konstanthaltung der Pfropfenbildung oder zur Ansteuerung von Stopp-, Alarm- oder Warnvorrichtungen oder gleichzeitig für die Regulierung und die Ansteuerung weiterverarbeitet werden.

Die entsprechende Vorrichtung umfasst eine Texturierdüse mit einem Förderkanal mit einer Einlassöffnung für einen Faden, mit einem Einlass für ein Fördermedium und mit einer Austrittsöffnung für Faden und Fördermedium und eine Stauchkammer, dadurch gekennzeichnet, dass im Bereich der Austrittsöffnung sensorische Mittel zur Überwachung dieses Bereiches vorhanden sind.

Letztere Erfindung beruht auf einer kontinuierlichen und automatischen Kontrolle der Pfropfenbildung die zu Regulierungs- oder Alarmzwecken ausgenützt wird. Diese erfolgt durch sensorische Überwachung des Bereiches der Austrittsöffnung, beispielsweise durch Messung des statischen Druckes oder einer mit dem statischen Druck korrelierten Grösse im Förderkanal nahe der Austrittsöffnung oder unmittelbar ausserhalb der Austrittsöffnung oder durch optische Überwachung der Stauchkammer nahe der Austrittsöffnung und durch Weiterverarbeitung der durch die sensorische Überwachung erhaltenen Signale zu Regel-, Steuer- und/oder Alarmzwecken.

Der statische Druck im Förderkanal ist die Differenz zwischen dem Gesamtdruck, der im wesentlichen konstant ist, und dem dynamischen Druck, der zum Quadrat der Strömungsgeschwindigkeit proportional ist. Bei leerem Förderkanal (ohne Faden), durch den das Fördermedium durch keinen Faden gehindert strömen kann, ist der statische Druck am geringsten, da die Strömungsgeschwindigkeit hoch ist. Fördert das Medium einen Faden durch den Förderkanal, ist der stationäre Druck verglichen mit dem stationären Druck im fadenfreien Kanal höher, weil das Medium sich am Faden staut. Entsteht vor der Austrittsöffnung in der Stauchkammer ein Pfropfen, wird das Medium noch stärker gestaut und der statische Druck wird entsprechend höher. Dabei ist der statische Druck umso höher, je näher an der Austrittsöffnung die Pfropfenbildung beginnt. Die Messung des statischen Druckes im Förderkanal nahe der Austrittsöffnung kann also direkt als Angabe für den Zustand der Pfropfenbildung ausgewertet werden.

Etwa dasselbe gilt für den statischen Druck in der Stauchkammer, unmittelbar ausserhalb der Austrittsöffnung.

In derselben Weise kann die Pfropfenbildung durch optische Sensoren in der Stauchkammer überwacht werden. Für eine gute Kräuselung muss die Pfropfenbildung möglichst nahe an der Austrittsöffnung, höchstens um einen vorgegebenen empirisch ermittelten Abstand davon entfernt, beginnen. Entfernt sich der Pfropfenanfang weiter von der Austrittsöffnung, handelt es sich um einen Ausblaser. Mit einem optischen Sensor, der die Stauchkammer im Bereiche des maximal tolerierbaren Abstandes des Pfropfens von der Austrittsöffnung überwacht, kann ein Ausblaser festgestellt werden.

Da die Pfropfenbildung von der Bremsung in der Stauchkammer und von der Pfropfenweiterförderung abhängig ist, kann sie konstant gehalten werden durch Regulierung dieser Grössen. Das heisst die Überwachung der Pfropfenbildung, insbesondere die Überwachung des Druckes im Bereiche der Austrittsöffnung kann als Messglied in einen Regelkreis mit einem Proportional/Integral-Regler integriert werden, dessen Stellglieder die Bremswirkung der Stauchkammerwandungen und/oder die Weiterförderung, insbesondere die Weiterförderungsgeschwindigkeit des Pfropfens beeinflussen.

CH 2059/92 konzentriert sich dementsprechend auf die Betriebsverhältnisse in der Düse.

Diese Erfindung beruht auf der Erkenntnis, dass die Betriebsverhältnisse in einer Texturierdüse komplexer sind, als in CH 2059/92 erkannt wird. Sie können durch Betriebsparameter ausserhalb der Düse beeinflusst werden und Betriebsparameter ausserhalb der Düse selbst beeinflussen. Die Pfropfenbildung kann daher durch Abtastung vom Betriebsparameter an einer Stelle festgestellt werden, die einen Abstand vom Pfropfen selbst aufweist.

Es sind aber auch beim Texturieren weitere Stellgrössen zur Beeinflussung der Ergebnisse möglich, als in EP-554642A1 vorgeschlagen wurden. Insbesondere wird nun vorgeschlagen, dass der Druck und/oder die Temperatur des Texturiermediums (der Förderluft), auch "Speiseluft" genannt, als Stellgrösse verwendet werden soll.

Das erfindungsgemässe Verfahren und beispielhafte Ausführungsformen der erfindungsgemässen Vorrichtung sollen nun anhand der folgenden Figuren detailliert beschrieben werden, wobei vom System nach CH 2059/92 ausgegangen wird. Es zeigen:
- Fig. 1: ein Schema der Vorrichtung nach EP-554642A1.
- Fig. 2: den Verlauf des Messsignales einer Druckmessung im Förderkanal beim Anfahren der Vorrichtung nach Fig.1 und während ihrem Betrieb,
- Fig. 3: den Verlauf des Messsignales einer Druckmessung im Förderkanal mit Regelkreis, Warnband und Alarmband,
- Fig.4: den Verlauf des Messsignales eines optischen Sensors in der Stauchkammer beim Anfahren der Vorrichtung nach Fig. 1 und während ihrem Betrieb,
- Fig. 5a und 5b: eine Texturierdüse mit Stauchkammer mit einer Messöffnung zur Messung des statischen Druckes im Förderkanal und Mitteln zur optischen Überwachung der Stauchkammer,
- Fig. 6: eine Texturierdüse mit Stauchkammer mit Mitteln zur Überwachung des Druckes in der Stauchkammer,
- Fig. 7: eine schematische Darstellung (ähnlich Fig. 1) einer ersten Ausführung der nun vorliegenden Erfindung,
- Fig. 8: eine weitere schematische Darstellung einer zweiten Ausführung, wobei in dieser Ausführung die Überwachung vom Betriebsparameter der Düse selbst durch eine Überwachung ergänzt oder sogar ersetzt wird, die auf Betriebsparameter ausserhalb der Düse anspricht, und
- Fig. 9 und 11: je eine Modifikation der Ausführung nach der Fig. 8.
- Fig. 10: eine Modifikation der Ausführung nach Fig. 7.
- Fig. 12: eine schematische Darstellung einer weiteren Ausführungsform
- Fig. 13 und 14: je eine Modifikation der Ausführung nach der Fig. 12

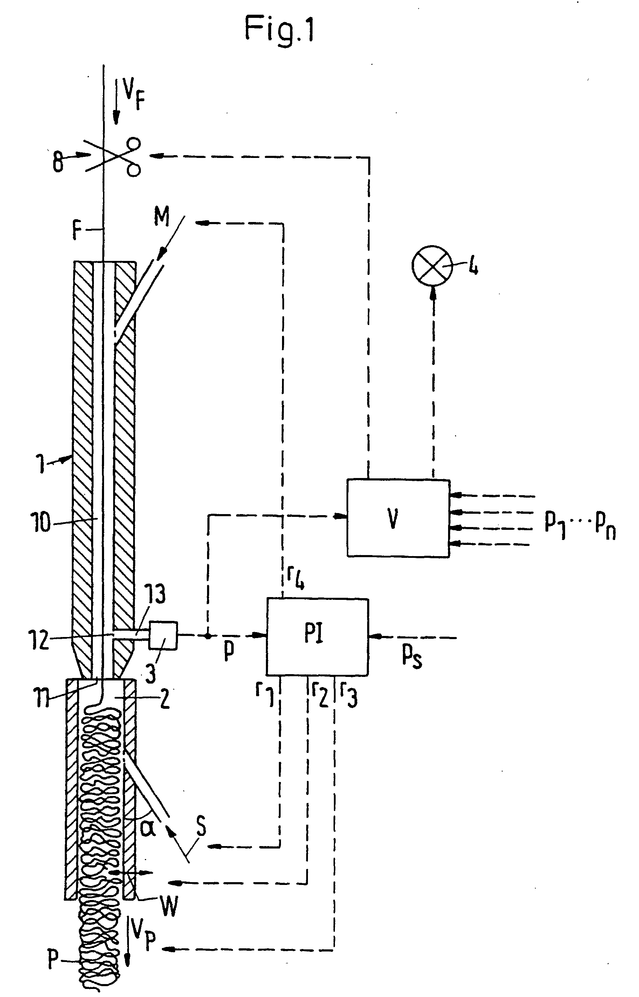

Fig. 1 zeigt ein Schema der Vorrichtung nach EP-554642A1, anhand dessen das Verfahren nach der gleichen Anmeldung erläutert werden soll. Die Vorrichtung weist einen Düsenteil 1 mit einem Förderkanal 10 und einer Austrittsöffnung 11 auf und eine an diese anschliessende Stauchkammer 2, die entlang dem Verlauf eines zu texturierenden Fadens F geschnitten dargestellt sind. Der Faden F wird mit Hilfe eines Fördermediums M, das unter Druck in den Förderkanal eingespeist wird, mit der Fadengeschwindigkeit V_{F} durch den Förderkanal 10 und aus der Austrittsöffnung 11 gefördert. Dabei hat das Fördermedium eine erhöhte Temperatur, um den Faden gleichzeitig zu erhitzen. Das Fördermedium M wird unter Druck in den Förderkanal 10 eingespeist und entspannt sich ausserhalb der Austrittsöffnung 11. Der Faden F wird vom Fördermedium durch den Förderkanal gefördert und prallt ausserhalb der Austrittsöffnung auf den Pfropfen P, der mit einer Pfropfengeschwindigkeit Vₚ weitergefördert wird, um in weiteren Schritten abgekühlt und dann zu einem texturierten Faden aufgelöst zu werden.

In die Stauchkammer 2 kann mit einem Winkel α zur Pfropfenbewegungsrichtung ein Staumedium S unter Druck gegen den Faden eingespeist werden. Der Winkel α ist dabei zwischen 0 und 90° zu wählen, derart, dass die Strömungsrichtung des Staumediums keine Komponente in Richtung der Pfropfengeschwindigkeit aufweist. Das Staumedium S wird vor allem beim Anfahren aber auch je nach Bedarf während des Betriebes der Vorrichtung dazu benützt, durch zusätzliche Bremsung die Pfropfenbildung zu initiieren bzw. zu gewährleisten, indem durch das Staumedium der Faden gebremst und gegen die Wandungen der Stauchkammer bewegt und dadurch zusätzlich durch Reibung an diesen Wandungen gebremst wird.

In der Wandung des Förderkanales ist eine Messöffnung 12 angebracht, an die sich ein Messhohlraum 13 anschliesst. Der Messhohlraum 13 ist bis auf die Messöffnung geschlossen und weist ein Druckmesselement 3, beispielsweise ein Piezoelement auf, mit dem der Druck im Messhohlraum 13 gemessen wird, der dem statischen Druck im Förderkanal (Bereich der Messöffnung) entspricht.

Der Messwert p des Druckmesselementes 3, der dem statischen Druck im Förderkanal entspricht, wird als Messgrösse in einen Proportional/Integral-PI-Regler gespeist und/oder in eine Vergleichereinheit V. Mit dem Ausgangssignal (r₁, r₂, r₃, oder r₄) des Reglers Pl kann entweder die aerodynamische Bremswirkung in der Stauchkammer durch Regulierung der Zufuhr des Staumediums S(r₁), oder die mechanische Reibung in der Stauchkammer durch Regulierung W der Stauchkammerwandung oder Stauchkammergeometrie (r₂), oder die Pfropfengeschwindigkeit durch Regulierung der Geschwindigkeit eines an die Stauchkammer anschliessenden Pfropfenfördermittels (r₃), oder die Stauchwirkung durch Regulierung der Zufuhr des Fördermediums M (r₄) derart beeinflusst werden, dass der Staudruck p einem Sollwert pₛ entspricht. Der Solldruck pₛ kann durch Versuche ermittelt und in den Regler eingegeben werden oder durch eine Eichmessung bestimmt werden.

Die Stellglieder (in der Figur nicht dargestellt) für den Regelkreis sind beispielsweise Regulierventile für die Zufuhr des Staumediums oder des Fördermediums, ein Antrieb, mit dem ein Bremskörper in die Stauchkammer bewegt oder die Stauchkammer irisähnlich verengt wird oder der Antrieb eines gegebenenfalls vorgesehenen an die Stauchkammer anschliessenden Pfropfenfördermittels. Die Regulierung der Zufuhr des Staumediums eignet sich vor allem in Vorrichtungen mit Stauchkammern mit teilweise sich bewegenden Wandungen, die Regulierung der Stauchkammerwandung oder deren Geometrie (beispielsweise der Konizität der Stauchkammer) eignet sich vor allem für Vorrichtungen mit nur stationären Stauchkammerwandungen, wobei die Grössenordnungen der notwendigen Veränderungen sehr klein (im Bereiche von Zehntelsmillimetem) sind. Eine Iris-artige Bewegung ist vor allem für aus einzelnen, stationären Lamellen gebildeten Stauchkammern günstig. Die Regulierung der Pfropfenfördergeschwindigkeit ist nur möglich, wenn die Kräuselvorrichtung ein an die Stauchkammer anschliessendes Pfropfenfördermittel, beispielsweise eine Nadelwalze, aufweist.

Nach der Lehre der EP-554642A1 ist die Regulierung der Zufuhr des Fördermediums weniger vorteilhaft, da sie sich auch auf die Fadentemperatur und dadurch auf die Kräuselung auswirken wie aber nachfolgend näher erläutert wird, ist es nun vorgesehen, diese Grössen zu beeinflussen.

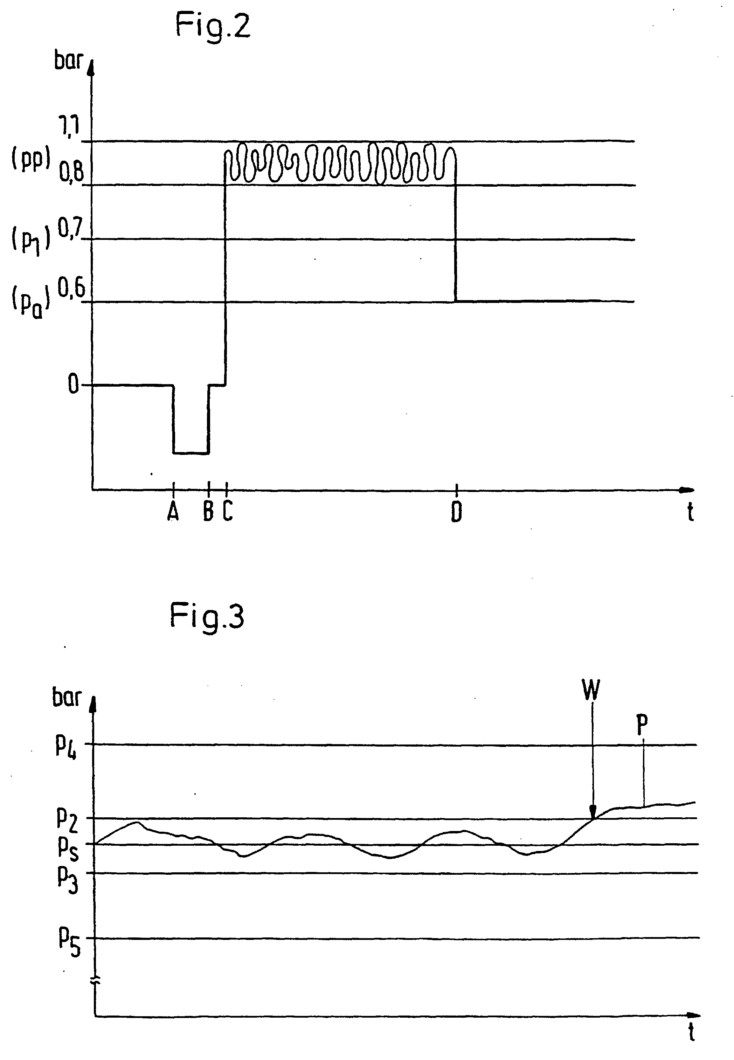

Der durch die Druckmessung erzeugte Messwert kann auch in einer Vergleichereinheit V mit mindestens einem Grenzmesswert (p₁...pₙ) verglichen werden. Werden Grenzwerte überschritten, kann beispielsweise eine Alarmleuchte 4 angesteuert werden oder die Produktion durch Fadenunterbruch (8) gestoppt werden. Die Funktion der Vergleichereinheit soll noch im Zusammenhang mit den Figuren 2, 3 und 4 detailliert beschrieben werden.

Für die Funktion des Reglers Pl kann ein handelsüblicher Proportional/Integral-Regler eingesetzt werden. Für eine kombinierte Regel- und Vergleichs-Funktion kann ein Integralregler beispielsweise mit Alarm- und Stoppband eingesetzt werden. Soll nur verglichen und nicht geregelt werden, kann für die Funktion des Vergleichers V beispielsweise eine Schaltung mit einem Diskriminator mit einstellbarem Schwellenwert eingesetzt werden. Die Schwellenwerte werden dabei durch Versuche eingestellt. Selbstverständlich kann die Regel- und/oder Vergleichsfunktion auch softwaremässig realisiert werden.

Figuren 2, 3 und 4 zeigen nun beispielhafte Verläufe eines Messsignals einer Überwachung nach EP-554642A1. Auf der Ordinate ist das Messsignal bzw. die dem Messsignal entsprechende physikalische Grösse aufgetragen, auf der Abszisse die Zeit.

Fig. 2 zeigt den Messsignalverlauf für eine Anordnung mit Druckmessung im Förderkanal und Auswertung des Messignales mit einer Vergleichereinheit. Der statische Druck (in bar Über- bzw. Unterdruck) im Förderkanal, der dem Messwert p (beispielsweise elektrische Spannung) entspricht ist dabei über einer Zeitachse t aufgetragen, wobei die dargestellte Zeit das Anlaufen der Vorrichtung, stationären Betrieb und das Auftreten eines Ausblasers enthält.

Bis zum Zeitpunkt A strömt durch den Förderkanal kein Fördermedium, wobei dieser geschlossen oder für Vorbereitungsarbeiten offen sein kann, das heisst entlang dem Fadenlauf in zwei Kanalteile getrennt ist. Bis zu diesem Zeitpunkt ist der statische Druck in der Düse gleich Atmosphärendruck, der gemessene Druck also gleich Null. Zum Zeitpunkt A wird der Förderkanal geschlossen und das Fördermedium eingeschaltet, so dass es den Kanal durchströmt. Der statische Druck im Kanal sinkt und bleibt während der folgenden Aufheizzeit konstant. Wenn der Kanal Texturiertemperatur erreicht hat (Zeitpunkt B), wird das Fördermedium wieder gestoppt, der Kanal geöffnet und der Faden eingezogen. Zum Zeitpunkt C wird der Kanal geschlossen und sofort die Pfropfenbildung initiiert, beispielsweise durch kurzzeitige Fadenbremsung mit Hilfe des Staumediums S. Durch die Stauung des Fördermediums am Faden im Förderkanal und am Pfropfen in der Stauchkammer steigt der statische Druck im Förderkanal und pendelt sich bei kontinuierlichem und regelmässigem Betrieb in einem Druckbereich entsprechend einem Messwertbereich pp ein. Der Betrieb kann als optimal bezeichnet werden, wenn der Messwertbereich pp möglichst schmal ist und über lange Zeiträume konstant bleibt.

Im Zeitpunkt D tritt nun ein Ausblaser auf, das heisst die Stelle der Pfropfenbildung entfernt sich von der Austrittsöffnung. Dadurch wird die Stauwirkung des Pfropfens kleiner und der gemessene statische Druck fällt, und zwar auf einen Druck entsprechend einem Messwert pₐ, der im Extremfall dem statischen Druck im Förderkanal ganz ohne Stauwirkung eines Pfropfens entspricht.

Mit einer Messung in der Art nach Fig. 2, wobei der Messwert p verfolgt und die Pfropfenbildung beobachtet wird, kann ein Schwellenmesswert p₁ bestimmt werden, derart, dass Unregelmässigkeiten in der Pfropfenbildung soweit tolerierbar sind, solange sie keine Messwertschwankungen bedingen, die zu einer Unterschreitung des Schwellenmesswertes p₁ führen. Der Schwellenmesswert p₁ wird höher angesetzt als der Messwert pₐ für einen Ausblaser. Der Schwellenmesswert p₁ wird genügend tief angesetzt, dass er einen genügenden Abstand vom Messwertbereich pp hat, derart, dass bei regelmässigem Betrieb die Messwerte p nicht in seinen Bereich fallen. Der Schwellenmesswert p₁ wird genügend hoch angesetzt, dass Pfropfenunregelmässigkeiten, die zu Qualitätseinbussen und/oder zu permanenten Ausblasern führen, als solche erkannt werden.

Für eine Vorrichtung entsprechend der eingangs bereits erwähnten Kräuselvorrichtung analog der europäischen Patentschrift Nr. 310890 ergaben sich beispielsweise folgende Druckverhältnisse: Bei einem Speisedruck des Fördermediums von 7 bis 7,5 bar lag der Druckbereich bei regelmässiger Pfropfenbildung (Messwertbereich pp) zwischen 0,8 bis 1,1 bar (Überdruck), der gemessene Druck bei einem Ausblaser (Messwert pₐ) betrug 0,6 bar, so dass der Schwellendruck (Schwellenmesswert p₁) bei ca. 0,7 bar angesetzt werden musste.

Fig. 3 zeigt einen beispielhaften Signalverlauf für eine Anordnung mit Messung des Druckes im Förderkanal, mit Regelkreis, Warnband (p₂p₃) und Alarmband (p₄/p₅). Bei optimalem Betrieb sollte der geregelte Messwert innerhalb des Warnbandes liegen. Liegt der Messwert ausserhalb des Warnbandes, aber noch innerhalb des Alarmbandes kann noch ohne Qualitätseinbusse produziert werden aber es wird eine Warnung W erzeugt (Warnleuchte, Protokollausdruck), die auf eine bald notwendige Revision (Reinigung) hinweist. Steigt der gemessene Druck über p₄ ist die Austrittsöffnung verstopft, sinkt er unter p₅, liegt ein Ausblaser vor. In beiden Fällen muss die Produktion beispielsweise durch Fadenschnitt gestoppt werden.

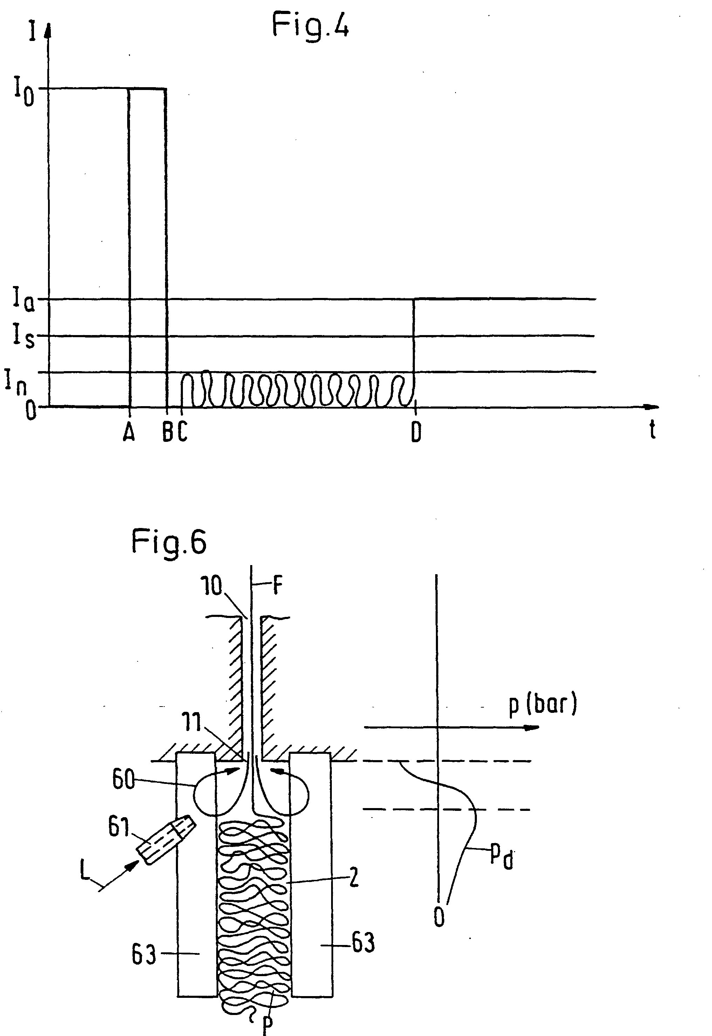

Fig. 4 zeigt den Verlauf des Messsignales I eines optischen Sensors in der Stauchkammer über den gleichen Zeitverlauf aufgetragen wie Fig. 2. Das Messsignal I ist beispielsweise das Messsignal eines optischen Sensors bestehend aus Lichtquelle und lichtempfindlicher Zelle, die einander gegenüber liegend in der Stauchkammer angebracht sind. Das von der Lichtquelle emittierte Licht ist gegen die lichtempfindliche Zelle gerichtet, wird aber von Faden und/oder Pfropfen teilweise absorbiert und gestreut. Das Messsignal entspricht der Intensität des von der lichtempfindlichen Zelle empfangenen Lichtes. Diese ist hoch bei fadenloser Stauchkammer (I₀), niedriger bei im wesentlichen gradlinig durchlaufendem Faden (Iₐ) bedingt durch Absorption durch den Faden, was einem Ausblaser entspricht, und sehr niedrig bei einem Pfropfen in der Stauchkammer (Bereich IHn). Der Schwellenmesswert Iₛ wird angesetzt zwischen Iₐ und der oberen Grenze von Iₙ.

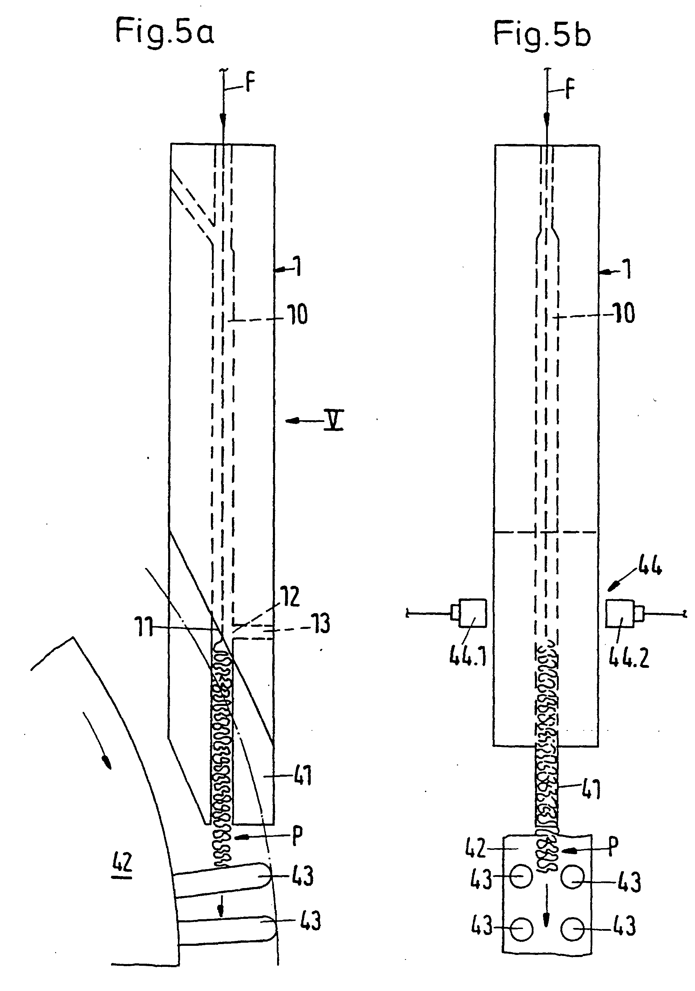

Fig. 5a und 5b zeigen als zwei beispielhafte Ausführungsformen der Kräuselvorrichtung nach EP-554642A1 je einen Düsenteil 1 mit Anformungen 41, die die Funktion der Stauchkammer übernehmen. Die Texturierdüse ist in der Fig. 5b gegenüber der Fig. 5a um 90° gedreht (Blickrichtung wie Pfeil V in Fig. 5a). Der Faden F wird wie beschrieben durch den Förderkanal 10 und durch die Austrittsöffnung 11 gefördert. Unmittelbar ausserhalb der Austrittsöffnung beginnt die Pfropfenbildung. Der gebildete Pfropfen P wird mit Hilfe einer Pfropfenförderwalze 42 zwischen Zähnen 43 weg gefördert.

In der Vorrichtung gemäss Fig. 5a wird der Staudruck im Förderkanal gemessen. Die Vorrichtung zeigt eine Messöffnung 12, die in einen Messhohlraum 13 führt. Der Messhohlraum kann ausserhalb der Wandung des Förderkanales beliebige, der Gesamtanordnung angepasste Formen annehmen. Das Druckmesselement (in der Fig. nicht dargestellt) ist vorteilhafterweise ausserhalb der Förderkanalwandungen angebracht.

In der Vorrichtung gemäss Fig. 5b wird die Pfropfenbildung optisch überwacht. Die Vorrichtung zeigt eine Lichtschrankenanordnung 44, die alternativ zu Messhohlraum und Druckmesselement vorgesehen sein kann. Es handelt sich beispielsweise um eine Lichtquelle 44.1 und einen Empfänger 44.2, die einander gegenüberliegend auf den offenen Seiten der Stauchkammer derart angebracht sind, dass der Empfänger das Licht der Lichtquelle empfängt.

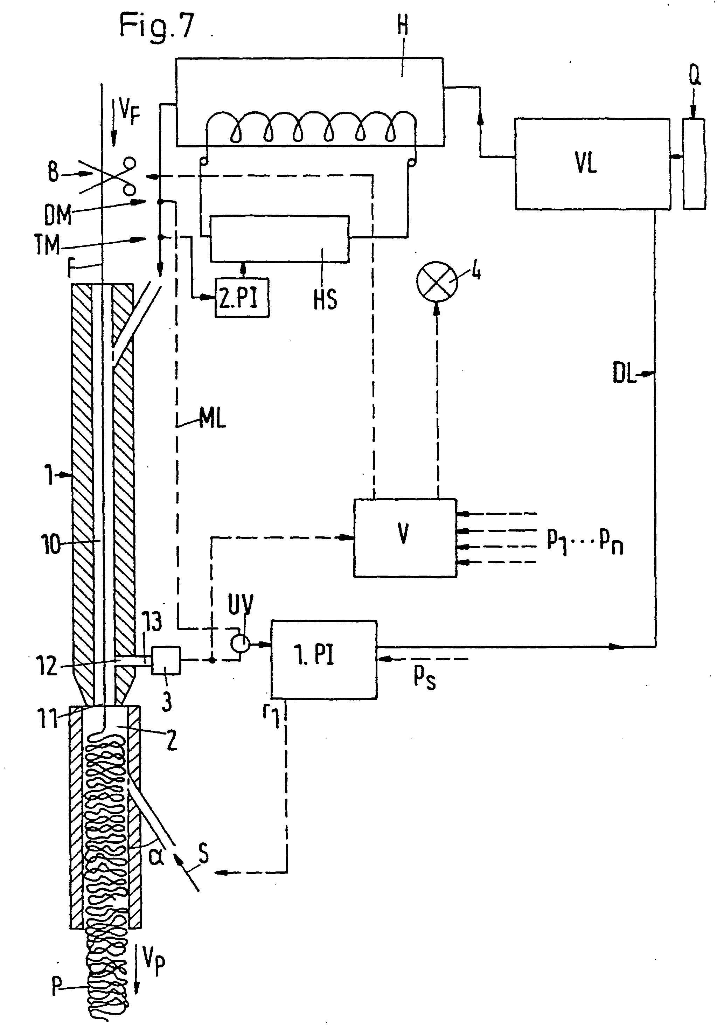

Fig. 6 zeigt schematisch eine weitere beispielhafte Anordnung zur Überwachung des Druckes im Bereiche der Austrittsöffnung. Es handelt sich um die Messung des dynamischen Druckes in einer Zuleitung für Messluft in die Stauchkammer und zwar unmittelbar ausserhalb der Austrittsöffnung.

Die Figur zeigt wiederum einen Förderkanal 10, in dem ein Faden F gefördert wird und eine Stauchkammer 2, in der sich ein Pfropfen P bildet. Die Stauchkammer 2 ist in der dargestellten beispielhaften Ausführungsform begrenzt durch radial zum Pfropfen angeordnete Lamellen 63. Im Bereiche der Austrittsöffnung 11 entspannt sich das Fördermedium zwischen die Lamellen. Messungen zeigen, dass sich zwischen Pfropfen P und Austrittsöffnung 11 Wirbel (Pfeile 60) bilden, derart, dass nahe an der Austrittsöffnung eine Strömung gegen den Faden, nahe am Pfropfen eine Strömung aus der Stauchkammer entsteht. Die Form dieser Wirbel ist stark von der geometrischen Ausgestaltung von Austrittsöffnung und Stauchkammer abhängig.

Wird nun im Bereiche zwischen Austrittsöffnung und Pfropfenanfang mittels einer fluidischen Düse der Druck an der Staukammerwand in Abhängigkeit der Distanz von der Austrittsöffnung gemessen, so ergibt sich, wie neben dem Vorrichtungsschema der Fig. 6 dargestellt, unmittelbar ausserhalb der Austrittsöffnung ein Unterdruck (Sog), der über eine neutrale Zone zu einem Druckmaximum beim Pfropfenanfang ansteigt. Wird nun beispielsweise in dem Abstand von der Austrittsöffnung, in dem bei optimaler Produktion der Pfropfen beginnt, eine derartige fluidische Düse 61 installiert, kann aus dem in der Düse gemessenen Druck eine Aussage gemacht werden über die Position des Pfropfens. Ein derartiges Messsignal kann in analoger Weise zum Messsignal des Sensors für den Staudruck im Förderkanal ausgewertet werden.

Unter fluidischer Düse wird eine Messdüse verstanden, durch die mit konstanter Leistung Messluft strömt und in der der Staudruck gemessen wird. Eine derartige fluidische Düse erweist sich im Bereiche der Stauchkammer als besonders vorteilhaft, da sie dank dem Messluftstrom in hohem Grade selbstreinigend ist.

Vorteilhafterweise ist der Messhohlraum bzw. die Mittel zur Überwachung der Stauchkammer, beispielsweise die Lichtschranke, so nahe wie möglich an der Austrittsöffnung vorgesehen.

Fig. 7 zeigt eine erste Erweiterung nach der nun vorliegenden Erfindung, wobei die Bezugszeichen nach Fig. 1 nochmals verwendet wurden, um die gleichen Teile anzudeuten. Die Ergänzungen nach Fig. 7 umfassen eine Quelle Q von Druckluft, ein steuerbares Ventil VL um den Druck von Luft aus der Quelle Q zu beeinflussen, ein Heizer H für die vom Ventil VL gelieferte Luft und ein Steuergerät HS für den Heizer H. Der Zustand der Luft stromabwärts vom Heizer H wird bezüglich Druck über den schon bezüglich Fig. 1 genannten ersten PI-Regler und bezüglich Temperatur über einen weiteren, zweiten Pl-Regler gesteuert und diese Luft wird als Speiseluft, das heisst als Förder- bzw. Texturiermedium an die Vorrichtung 1 geliefert.

Der erste PI-Regler ist mit dem Ventil VL über die Leitung DL, und der zweite PI-Regler mit dem Steuergerät HS über die Leitung HL verbunden. Über die Leitung DL kann der Luftdruck stromabwärts vom Ventil VL als Stellgrösse verwendet werden, um den vorerwähnten Staudruck "P" als Regelgrösse innerhalb vorgegebenen Toleranzen zu halten.

Es können sowohl ein handelsübliches Druckmessgerät DM als auch ein handelsübliches Temperaturmessgerät TM zwischen dem Heizer H und dem Einlass der Speiseluft in die Vorrichtung 1 vorgesehen werden. Das Ausgangssignal vom Gerät DM wird über eine Leitung ML an den ersten Pl-Regler weitergeleitet, so dass dieser Druck innerhalb vorgegebener Grenzen gehalten werden kann, während das Ausgangssignal des Gerätes TM über eine Leitung TL an den zweiten Pl-Regler geleitet wird, welcher sein Signal über eine Leitung HL an das Steuergerät HS abgibt.

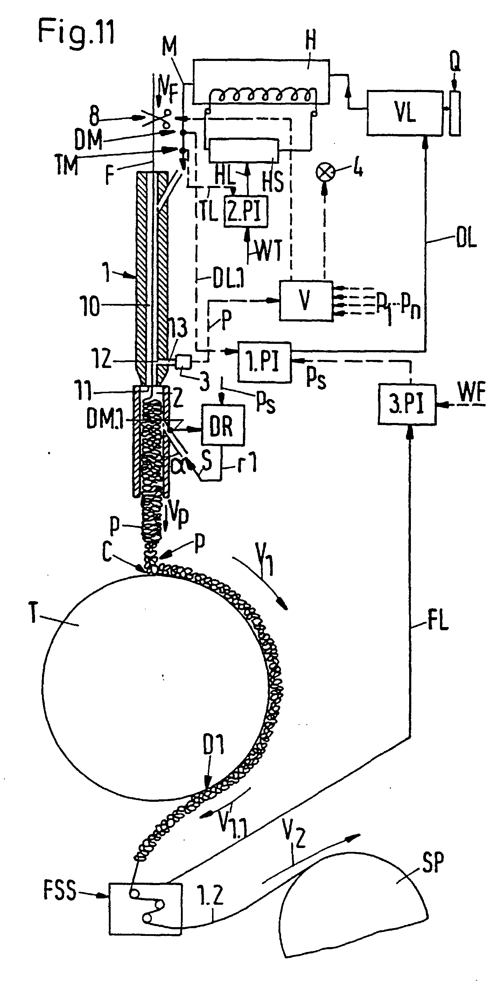

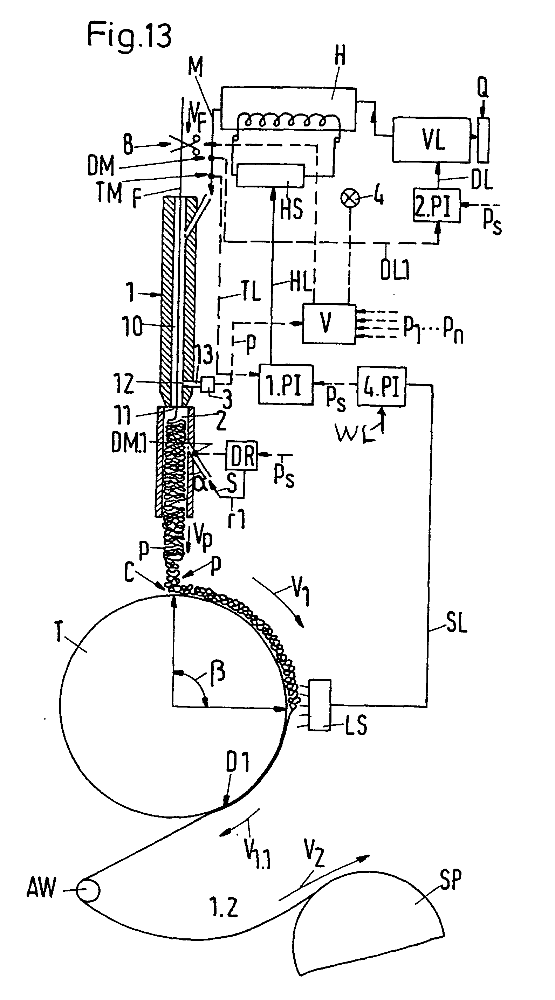

Dabei wird entweder der Staudruck P oder das Drucksignal der Leitung ML an den ersten Pl-Regler eingegeben, was mittels eines handelsüblichen Umschaltventiles UV ausgeführt wird. Hingegen wird der Staudruck P permanent der Vergleichereinheit V zugeführt. Die Ausgangssignale des ersten Pl-Regler sind entweder das Signal r1 zur Regulierung des Staumediums S oder das Signal für das steuerbare Ventil VL. Das Staumedium S kann jedoch auch eine unabhängige Regelung aufweisen (siehe Fig. 11, 13 u. 14).

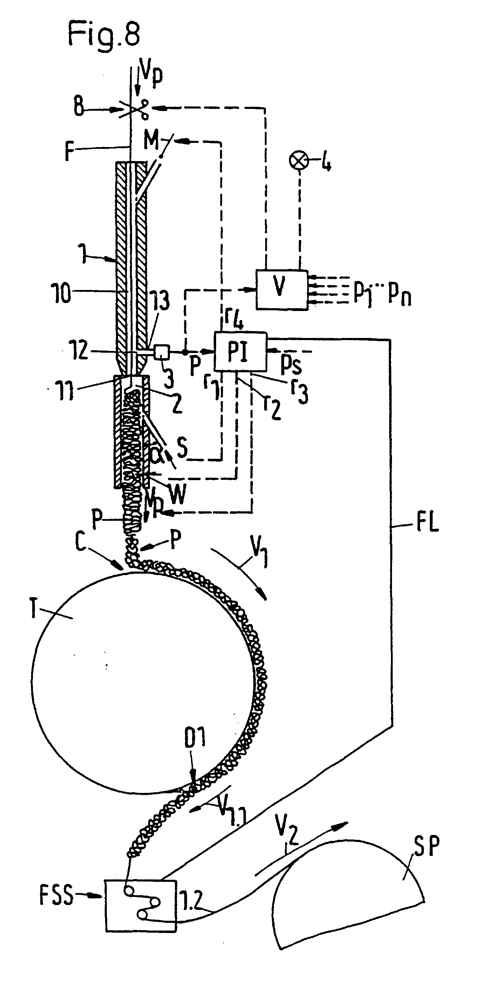

Fig. 8 zeigt eine Kopie der Fig. 1 ergänzt durch die folgenden neuen Elemente:
- eine rotierbare Kühltrommel T, welche den Pfropfen P übernimml.
- einen Sensor FSS, welcher die Fadenzugkraft, auch Fadenspannung genannt, nach dem Abzug von der Kühltrommel misst.

Der Sensor FSS liefert sein Ausgangssignal über eine Leitung FL an den PI-Regler und beeinflusst dadurch die Betriebsparameter, die anhand der Fig. 1 erklärt wurden, um die vom Sensor FSS gemessene Fadenzugkraft in vorgegebenen Grenzen zu halten.

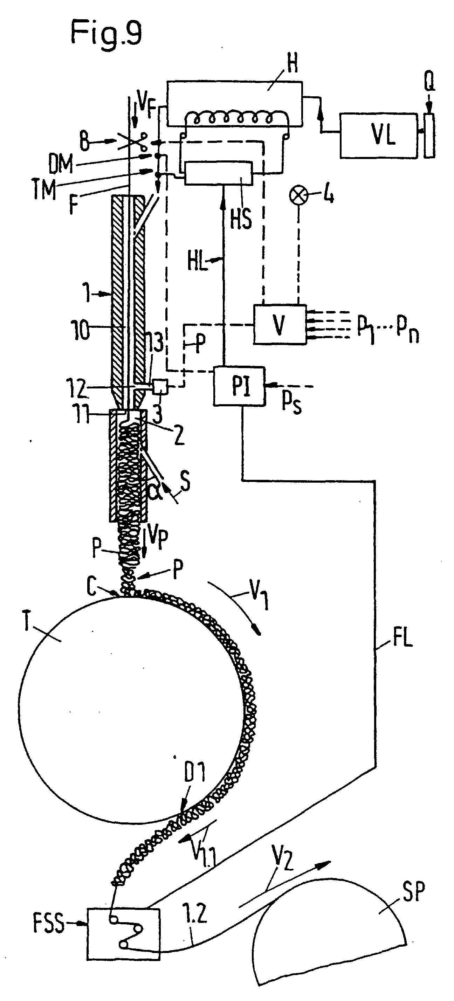

Bei den nach folgenden Figuren 8 und 9 handeltes sich nicht um Ausfünrungsbeispiele der Erfindung sondern um Beispiele die das Verständnis der Erfindung erleichtetern.

Die Kühltrommel T der Fig. 8 wird nach EP 0 488 939 gebildet. Der Pfropfen P, der aus der Stauchkammer austritt, wird im Punkte C auf die mit konstanter Oberflächengeschwindigkeil V₁ rotierende Kühltrommel T, welche als Siebtrommel oder perforierte Walze gebaut ist, aufgebracht. In die Kühltrommel T wird Luft gesaugt, die gleichzeitig den Pfropfen P auf der Trommeloberfläche festhält und kühlt. Der Pfropfen P bewegt sich mit der Oberfläche der Trommel und wird, wenn er den Punkt D1 erreicht hat, durch eine entsprechende Schikane (nicht gezeigt) oder durch Schliessung der Perforation der Kohltrommel T von dieser abgehoben, so dass er durch den Unterdruck in der Trommel nicht mehr gehalten wird, d.h. von der Trommeloberfläche gelöst wird. Das Garn 1.2 wird von der Abzugsspule SP mit der Geschwindigkeit V₂ abgezogen.

Fig. 9 zeigt teilweise das Schema der Fig. 7 mit Ergänzungen entsprechend den vorerwähnten Ergänzungen in der Fig. 8. Diese Ausführung dient dem gleichen Zweck, wie die Ausführung nach Fig. 8, wobei in diesem Fall der Druck bzw. die Temperatur der Förder- bzw. Texturierluft beeinflusst werden, um die Fadenspannung konstant zu halten, indem das Signal des Druckmessgerätes DM über die Leitung ML dem PI-Regler und das Signal des Temperaturgerätes TM über die Leitung TL an das Steuergerät HS abgegeben wird.

Die Überwachung der Pfropfenbildung eröffnet aber weitere Möglichkeiten, die nach Weiterentwicklung des nun vorgeschlagenen Prinzipes erschlossen werden sollen. Die Pfropfenbildung wird durch drei Arten von Betriebsparametern beeinflusst:
1. Parameter, die einen Einfluss auf den Faden ausüben, der an die Vorrichtung 1 geliefert wird,
2. Die Betriebsparameter der Vorrichtung selbst, und
3. Die Betriebsparameter der anschliessenden Einrichtungen zum Beispiel der Kühltrommel.

Wenn es sich als möglich erweist, Fehler der einen Art auszuschliessen bzw. zu erkennen, dann können durch die Überwachung der Pfropfenbildung Fehler anderer Arten erkannt werden. Dazu kann sich es allenfalls als nützlich erweisen, Fehlererkennungssysteme anzuwenden, die in der Patentliteratur erwähnt worden sind zum Beispiel in DE-A-44 14 517.

Wie schon durch die Ausführungen nach Fig. 8 und 9 angedeutet wurde, beeinflusst die Texturiervorrichtung (die Pfropfenbildung) aber auch Betriebsparameter, die nach der Vorrichtung erscheinen zum Beispiel die Fadenzugkraft bzw. Fadenspannung, aber auch den Titer. Durch die Überwachung solcher Parameter ist es möglich, auf den Zustand der Texturiervorrichtung zu schliessen.

Bekannte Qualitätsüberwachungen, die auf der Messung der Fadenzugkraft beruhen, sind in den folgenden Patentschriften zu finden:
DE-A-41 19780
DE-A-44 13 549
US-C-4 685 629
EP-C-207 471

Bekannte Qualitätsüberwachungen, die auf der Messung des Garntiters beruhen, sind in den folgenden Patentschriften zu finden:
US-C-3 731 069
US-C-4 045 659
US-C-3 885 232
US-C-4 030 082
CH-C-551 923

Ergänzend sei noch erwähnt, dass das mit Bezug auf Fig. 1 auf Seite 9 oder mit Bezug auf Fig. 8 auf Seite 17 erwähnte Pfropfenfördermittel r₃ entweder die mit den Figuren 5a und 5b gezeigte und auf Seite 14 erwähnte Pfropfenförderwalze 42, im Zusammenhang mit dem in der europäischen Patentschrift Nr. 310890 gezeigten und beschriebenen Verfahren ist, oder eine hier nicht gezeigte Pfropfenförderwalze, welche an der Oberfläche beispielsweise mit Nadeln beschickt ist, um den Pfropfen aufzunehmen und beispielsweise einer mit den Figuren 8 und 9 gezeigten Kühltrommel T weiterzugeben, wobei die Pfropfengeschwindigkeit V_{P} mittels der genannten Walze 42 oder nicht gezeigten Walze, durch Variieren der Drehzahl mittels des PI-Reglers beeinflusst wird.

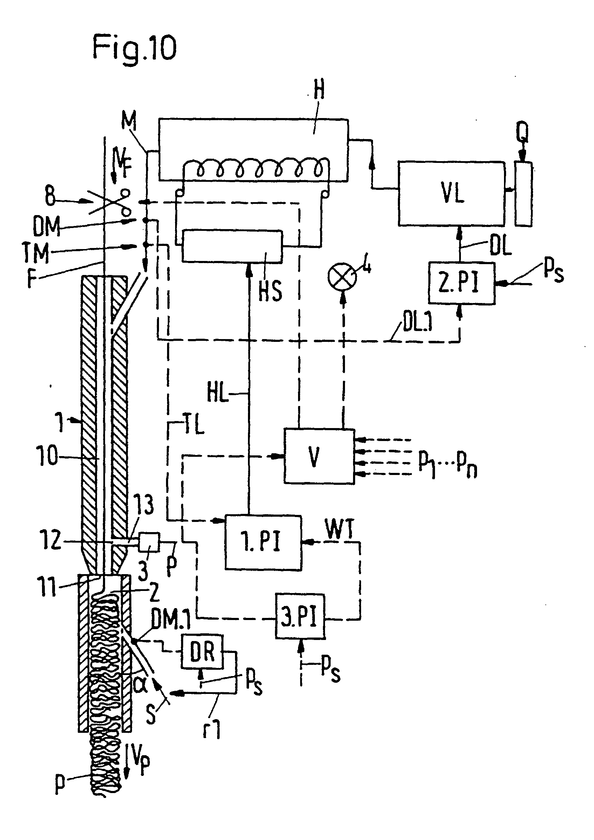

Die Figur 10 zeigt teilweise das Schema der Figur 7, dementsprechend sind die gleichen Elemente in Fig. 10 wie in Fig. 7 mit den gleichen Bezugszeichen versehen und werden in der Regel nicht nochmals beschrieben.

Die Fig. 10 zeigt einen ersten Regelkreis zur Regelung der Erwärmung und einen zweiten Regelkreis zur Regelung des Druckes des Fördermediums M.

Im ersten Regelkreis wird das Ausgangssignal eines ersten, mit einer Sollwerteingabe WT versehenen PI-Reglers an das Steuergerät HS mittels der Leitung HL zugeführt.

Im weiteren erhält der erste PI-Regler ein Temperatursignal mittels einer Leitung TL, vom Temperaturmessgerät TM.

Der Sollwert WT des ersten PI-Reglers wird von einem dritten, mit einer eigenen Sollwerteingabe PS versehenen PI-Regler dann geändert, wenn das Drucksignal p vom Druckmessgerät 3 nicht dem Sollwert PS entspricht, d.h., dass der Pfropfen in der Stauchkammer nicht die gewünschte Durchlässigkeit hat, sodass entsprechend die Temperatur des Fadens solange geändert wird, bis der Druck p dem Sollwert PS entspricht.

Der zweite Regelkreis weist einen zweiten, mit einer Sollwerteingabe PS versehenen PI-Regler auf, welcher den Druck im steuerbaren Ventil VL unter Einbezug des Signals des Druckmessgerätes DM, welches über die Leitung DL.1 dem zweiten PI-Regler zugeführt wird, regelt. Die Signale des zweiten PI-Reglers an das steuerbare Ventil VL werden in der Verbindungsleitung DL übermittelt.

Für das Staumedium S ist ein eigener Regelkreis vorgesehen, in dem das Signal eines am Einblasrohr für das Staumedium in die Staukammer vorgesehenes Druckmessgerätes DM.1, an einen mit einer Sollwerteingabe PS versehenen Druckregler DR abgegeben wird und der Druckregler DR den Druck des Staumediums S regelt.

Die Fig. 11 zeigt teilweise das Schema der Fig. 9, weshalb dieselben Elemente die gleichen Bezugszeichen aufweisen und nicht nochmals beschrieben werden.

Im weiteren weist die Fig. 11 einen ersten Regelkreis auf, um den Druck des Fördermediums in Abhängigkeit der im Fadenspannungsmessgerät FSS gemessenen Fadenspannung zu regein.

Ein zweiter und ein dritter, von der Fadenspannung unabhängiger Regelkreis regelt die Temperatur des Fördermediums, bzw. den Druck des Staumediums S.

Der erste Regelkreis weist einen ersten, mit einer Sollwerteingabe PS versehenen PI-Regler auf, welcher ein Ausgangssignal über eine Leitung DL an das steuerbare Ventil VL abgibt und ein Eingangssignal vom Druckmessgerät DM über eine Leitung DL.1 erhält.

Ein Signal des Fadenspannungsmessgerätes FSS wird über eine Leitung FL in einen dritten, mit einer Sollwerteingabe WF versehenen PI-Regler eingegeben, wobei falls das Fadenspannungssignal eine Differenz zum Sollwert WF aufweist, der dritte PI-Regler den Sollwert PS des ersten PI-Reglers so lange ändert, bis das Fadenspannungssignal dem Sollwert WF entspricht.

Der zweite Regelkreis beinhaltet einen zweiten, mit einer Sollwerteingabe WT versehenen PI-Regler, welcher einerseits ein Temperatursignal des Temperaturmessgerätes TM Ober eine Leitung TL erhält und ein Regelsignal über eine Leitung HL an das Steuergerät HS abgibt, welches den Heizer H steuert.

Ein vierter Regelkreis regelt die Anslauluft S, indem das Druckmessgerät DM.1 ein Drucksignal an einen mit einer Sollwerteingabe PS versehenen Druckregler DR abgibt und der Druckregler DR das Regelsignal r1 erzeugt, mittels welchem der Druck der Anstauluft S über ein hier nicht dargestelltes Ventil regelt.

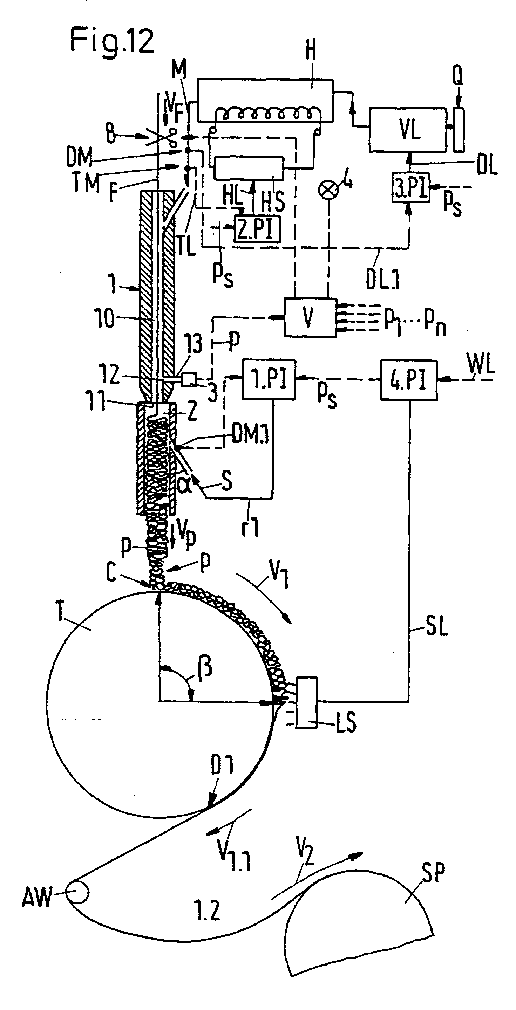

Die Figuren 12, 13 und 14 zeigen eine Variante betreffend der Beurteilung des sich auf der Kühltrommel T befindlichen Pfropfens, indem in diesen drei Figuren nicht die Fadenspannung zur Beurteilung des Pfropfens gemessen wird, sondern die Stelle auf der Kühltrommel T, an welcher der Pfropfen wieder zum Faden aufgelöst wird, welcher anschliessend von einer Abzugswalze AW abgezogen und der Spuleinrichtung SP weitergegeben wird.

Dabei wird diese vorgenannte Stelle auf der Kühltrommel mittels eines Lichtsensors LS festgestellt, wobei dieser Lichtsensor ein Lichtsender und Empfänger sein kann oder irgendein geeignetes Mittel zur Überwachung dieser Pfropfenauflösestelle und welches in der Lage ist, ein entsprechendes Signal abzugeben.

Das Signal des Sensors LS wird über eine Leitung SL in einen, mit einer Sollwerteingabe WL versehenen vierten bzw. dritten PI-Regler eingegeben, welcher einen Sollwert PS eines ersten PI-Reglers ändert, wenn das Signal SL nicht dem Sollwert WL entspricht, und zwar bis eine Übereinstimmung zwischen Signal SL und Sollwert WL herrscht.

In Fig. 12 erhält der erste Pl-Regler seinerseits ein Eingangssignal von einem Druckmessgerät DM.1, welches den Staudruck am Einblasrohr der Stauluft misst und das Staudrucksignal an den ersten PI-Regler abgibt. Andererseits gibt der erste PI-Regler das Drucksignal r1 ab, mittels welchem der Druck der Anstauluft S geregelt wird.

Ein zweiter Regelkreis mit einem zweiten, mit einer Sollwerteingabe PS versehenen PI-Regler regell die Temperatur des Fördermediums M.

Dazu erhält der zweite Pl-Regler ein Temperatursignal vom Temperaturmessgerät TM über eine Leitung TL und gibt ein Regelsignal über eine Leitung HL an das Steuergerät HS ab.

Ein dritter Regelkreis regelt den Druck des Fördermediums M, indem ein dritter, mil einer Sollwerteingabe PS versehener PI-Regler ein Drucksignal des Druckmessgerätes DM über eine Leitung DL.1 erhält und ein Regelsignal über eine Leitung DL an das steuerbare Ventil VL abgibt.

Im weiteren sei noch erwähnt, dass der Winkel β, welcher von der Stelle C, in welcher der Pfropfen auf die Kühltrommel gegeben wird, und der Stelle, an welcher der Pfropfen aufgelöst wird, begrenzt wird, Kühlwinkel β genannt wird.

Die Figur 13 zeigt teilweise das Schema der Fig. 12 auf, weshalb die gleichen Elemente mit denselben Bezugszeichen versehen sind, und in der Regel nicht nochmals beschrieben werden.

Die Fig. 13 zeigt drei Regelkreise und zwar einen ersten Regelkreis mit einem ersten PI-Regler. mittels welchem die Temperatur des Fördermediums in Abhängigkeit von der Stelle auf der Kühltrommel T, in welcher der Pfropfen aufgelöst wird, geregelt wird, und einen zweiten Regelkreis mit einem zweiten PI-Regler, mittels welchem der Druck des Fördermediums M geregelt wird, wie auch einem dritten Regelkreis mit einem Druckregler DR, mittels welchem die Anstauluft geregelt wird.

Im Zusammenhang mit dem ersten Regelkreis wird das Signal SL des Lichtsensors LS analog Fig. 12 einem vierten, mit einer Sollwerteingabe WL versehenen Pl-Regler eingegeben, wobei der vierte Pl-Regler die Sollwerteingabe PS des ersten PI-Reglers so lange ändert, bis das Signal SL dem Sollwert WL des vierten Pl-Reglers entspricht.

Dabei erhält der erste PI-Regler ein Temperatursignal des Temperaturmessgerätes TM über die Leitung TL und gibt ein Regelsignal über die Leitung HL an das Steuergerät HS ab, um den Heizer H zu steuern, welcher das Fördermedium M erwärmt.

Der zweite Regelkreis entspricht dem dritten Regelkreis der Fig. 12, lediglich mit dem Unterschied, das der Pl-Regler als zweiter PI-Regler gekennzeichnet ist. weshalb dieser Regelkreis nicht mehr beschrieben wird.

Der Regelkreis für die Anstauluft entspricht demjenigen der Fig. 10 bzw. 11.

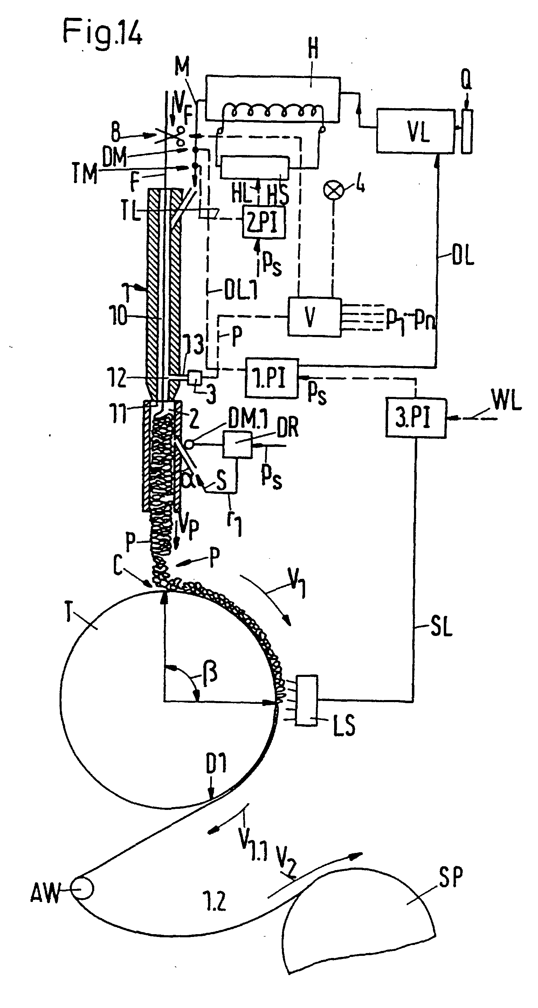

Die Fig. 14 weist teilweise dieselben Elemente auf, wie die Fig. 11, 12 und 13, weshalb die gleichen Elemente mit demselben Bezugszeichen versehen sind und nicht nochmals beschrieben werden.

Die Fig. 14 weist ebenfalls drei Regelkreise auf, wie die Fig. 13, wobei der erste Regelkreis nicht die Temperatur des Fördermediums M sondern den Druck in Abhängigkeit der Stelle regelt, an welcher der Pfropfen auf der Kühltrommel T aufgelöst wird.

Dementsprechend gibt der erste PI-Regler ein Regelsignal über die Leitung DL an das steuerbare Ventil VL ab, und erhält ein Eingangsignal vom Druckmessgerät DM über die Leitung DL.1.

Der erste PI-Regle weist eine Sollwerteingabe PS auf, welche von einem dritten Pl-Regler so lange verändert wird, bis das Signal vom Lichtsensor einer Sollwerteingabe WL des dritten PI-Reglers entspricht.

Der zweite Regelkreis beinhaltet den zweiten, mit einer Sollwerteingabe PS versehenen PI-Regler, welcher ein Temperatursignal vom Temperaturmessgerät TM über die Leitung TL erhält und ein Regelsignal über die Leitung HL dem Steuergerät HS abgibt.

Die Regelung der Anstauluft, welche den dritten Regelkreis bildet, ist dieselbe wie in Fig. 10, 11 und 13 beschrieben.

Es sei erwähnt, dass die Erfindung nicht auf die dargestellten Figuren eingeschränkt ist, sondern im Rahmen des Erfindungsgedanken, welcher eine vorgegebene Anzahl Stellgrössen und eine vorgegebene Anzahl Messgrössen enthält, die miteinander kombinierbar sind, nämlich die Stellgrösse zur Veränderung des Druckes des Fördermediums, die Stellgrösse zur Veränderung der Temperatur des Fördermediums, um den Faden zu erwärmen und die Stellgrösse zur Veränderung der Anstauluft, um die Stauung in der Stauchkammer mindestens zu starten sowie die Messgrösse, welche den Staudruck im Förderrohr angibt, die Fadenspannung des gekräuselten Fadens als weitere Messgrösse, sowie der Kühlwinkel β, bzw. die Stelle, an welcher der Pfropfen auf der Kühltrommel aufgelöst wird als dritte Messgrösse, wobei die Stellgrössen und die Messgrössen im Rahmen einer Matrix kombiniert werden, indem bspw. die Stellgrössen in der Abszisse und die Messgrössen in der Ordinate aufgeführt sind und die entsprechenden Kombinationen daraus entnommen werden.

## Patentansprüche

1. Verfahren zum kontinuierlichen Kräuseln eines Fadens, aus einem thermoplastischen Material, mit folgenden Verfahrensschritten;
- Komprimieren eines Fördermediums
- Erwärmen eines Fördermediums
- Fördern des Fadens mit einer Fördergeschwindigkeit durch einen Förderkanal (10) und durch eine Austrittsöffnung (11) des Förderkanales
- Fördern des Fadens von der Austrittsöffnung des Förderkanales in die Einlassöffnung einer Stauchkammer (2)
- Komprimieren des Fadens in der Stauchkammer zu einem Pfropfen durch Verzögern des Fadens
- Fördern des Pfropfens von einer Austrittsöffnung der Stauchkammer, mit einer Pfropfengeschwindigkeit, welche kleiner ist als die Fördergeschwindigkeit, derart, dass der Pfropfen gekühlt wird und
- Auflösen des Pfropfens zu einem gekräuselten jedoch gespannten Faden
- Mindestens einen von der Pfropfenbildung beeinflussten Parameter messen
- Vergleichen dieses einen gemessenen Parameters mit einem Sollwert für diesen Parameter
- und senden mindestens eines Signales an mindestens einen von diesem Parameter abhängigen Regelkreis, im Falle einer Differenz zwischen diesem gemessenen Parameter und dem Sollwert
- **dadurch gekennzeichnet,**
- **dass** es sich bei den abhängigen Regelkreisen um mindestens einen der folgenden Regelkreise handelt, nämlich
- um einen Regelkreis zur Regelung des vorgenannten Druckes des Fördermediums,
- einen Regelkreis zur Regelung der vorgenannten Temperatur des Fördermediums
- und einen Regelkreis zur Regelung der vorgenannten Bremsung des Fadens und,
- **dass** mindestens ein weiterer, von den vorgenannten Regelkreisen unabhängiger Regelkreis vorgesehen ist und zwar zur Regulierung mindestens
- des Druckes des Fördermediums,
- der Temperatur des Fördermediums und
- der Bremsung des Fadens
wenn diese letztgenannten Parameter nicht schon durch die abhängigen Regelkreise gesteuert sind.

2. Verfahren nach Anspruch 1, **dadurch gekennzeichnet, dass** es sich bei der gemessenen Grösse um den Druck des Fördermediums im Förderkanal (1) und beim einen abhängigen Regelkreis um den Regelkreis zur Regelung des vorgenannten Druckes des Fördermediums und beim anderen abhängigen Regelkreis um den Regelkreis zur Regelung eines in die Stauchkammer (2) eingeblasenen Staumediums (5) zur vorgenannten Bremsung des Fadens und beim unabhängigen Regelkreis um den Regelkreis zur Regelung der vorgenannten Temperatur des Fördermediums handelt.

3. Verfahren nach Anspruch 1, **dadurch gekennzeichnet, dass** es sich bei der gemessenen Grösse um eine Fadenspannung im gekräuselten jedoch gespannten Faden und beim abhängigen Regelkreis um den genannten Regelkreis zur Regelung des genannten Drucks des Fördermediums und, dass es sich beim unabhängigen Regelkreis je um einen Regelkreis zur Regelung der Bremsung des Fadens und um einen Regelkreis zur Regelung der Temperatur des Fördermediums handelt.

4. Verfahren nach Anspruch 3, **dadurch gekennzeichnet, dass** es sich beim unabhängigen Regelkreis zur Bremsung des Fadens um die Regelung einer in die Stauchkammer eingeblasenen Anstauluft (S) zum Anstauen des Pfropfens in der Stauchkammer handelt.

5. Verfahren nach Anspruch 1, **dadurch gekennzeichnet, dass** es sich bei der gemessenen Grösse um den statischen Druck im Förderkanal (10) im Bereich der Düsenaustrittsöffnung (11) und beim abhängigen Regelkreis um die Regelung der Temperatur des Fördermediums handelt und, dass es sich beim unabhängigen Regelkreis je um einen Regelkreis für die Regelung der Bremsung des Fadens und um einen Regelkreis zur Regelung des Druckes des Fördermediums handelt.

6. Verfahren nach Anspruch 5, **dadurch gekennzeichnet, dass** es sich beim genannten unabhängigen Regelkreis zur Bremsung des Fadens um den Regelkreis für die Regelung einer in die Stauchkammer eingeblasenen Anstauluft zum Anstauen des Pfropfens in der Stauchkammer handelt.

7. Verfahren nach Anspruch 1, **dadurch gekennzeichnet, dass** es sich bei der gemessenen Grösse um einen Kühlwinkel (β) an einer Kühltrommel (T) zur Kühlung des Pfropfens handelt und beim abhängigen Regelkreis, um den genannten Regelkreis zur Regelung des Drucks des Fördermediums handelt, in Kombination mit je einem unabhängigen Regelkreis für die Regelung der Bremsung des Fadens.und für die Regleung der Temperatur des Födermediums

8. Verfahren nach Anspruch 7, **dadurch gekennzeichnet, dass** es sich beim unabhängigen Regelkreis zur Bremsung des Fadens um den Regelkreis zur Regelung einer in die Stauchkammer eingeblasenen Anstauluft (S) zum Anstauen des Pfropfens in der Stauchkammer handelt.

9. Verfahren nach Anspruch 1, **dadurch gekennzeichnet, dass** es sich bei der gemessenen Grösse um einen Kühlwinkel (β) handelt und beim abhängigen Regelkreis um den genannten Regelkreis zur Regelung der Bremsung des Fadens handelt, in Kombination mit je einem unabhängigen Regelkreis zur Regelung des Drucks und der Temperatur des Fördermediums.

10. Verfahren nach Anspruch 9, **dadurch gekennzeichnet, dass** es sich beim abhängigen Regelkreis zur Bremsung des Fadens um den Regelkreis zur Regelung einer in die Stauchkammer eingeblasenen Anstauluft zum Anstauen des Pfropfens in der Stauchkammer handelt.

11. Verfahren nach Anspruch 1, **dadurch gekennzeichnet, dass** es sich bei der gemessenen Grösse um einen Kühlwinkel (β) an einer Kühltrommel zur Kühlung des Pfropfens und beim abhängigen Regelkreis um den Regelkreis zur Regelung der Temperatur des Fördermediums und beim unabhängigen Regelkreis um den Regelkreis zur Regelung des Druckes des Fördermediums und um den Regelkreis zur Regelung der Bremsung des Fadens handelt.

12. Verfahren nach Anspruch 11, **dadurch gekennzeichnet, dass** es sich beim Regelkreis zur Regelung der Bremsung des Fadens, um die Regelung einer in die Stauchkammer geblasene Anstauluft (S) zum Anstauen des Pfropfens in der Stauchkammer handelt.

13. Vorrichtung zum kontinuierlichen Kräuseln eines Fadens aus thermoplastischem Material, umfassend:
- ein Förderkanal (10) für einen Faden (F), mit einer Austrittsöffnung (11);
- eine Druckluftquelle (Q) für ein Fördermedium in Verbindung mit dem Förderkanal;
- eine Stauchkammer (2) mit einer Einlassöffnung und einer Auslassöffnung, in welcher der Faden zu einem Pfropfen gebildet wird;
- Kühlmittel zur Kühlung des Pfropfens;
- Mittel zur Auflösung des Pfropfens zu einem gekräuselten jedoch gespannten Faden;
- Mittel zur Messung mindestens eines Parameters des Pfropfens;
- Mittel zum Vergleichen dieses mindestens eines Parameters mit einem Sollwert und,
- Mittel zur Beeinflussung der Pfropfenbildung aufgrund dieses Vergleichs
**dadurch gekennzeichnet,**
**dass** die Mittel zur Beeinflussung der Pfropfenbildung mind. einen der folgenden, vom Resultat dieses Vergleichs abhängigen Regelkreise beinhalten:
- einen Regelkreis für das Regeln eines vorgegebenen Druckes im Fördermedium
- einen Regelkreis für das Regeln einer vorgegebenen Temperatur des Fördermediums und
- einen Regelkreis für die Regelung der Bremsung (S; W; VP) des Fadens in der Stauchkammer
und mindestens einen weiteren, von den vorgenannten Regelkreisen unabhängiger Regelkreis
- und zwar einen Regelkreis für das Regeln eines vorgegebenen Druckes im Fördermedium
- ein Regelkreis für das Regeln einer vorgegebenen Temperatur des Fördermediums und
- einen Regelkreis für die Regelung der Bremsung (S; W; Vp) des Fadens in der Stauchkammer.

14. Vorrichtung nach Anspruch 13, **dadurch gekennzeichnet, dass** die Mittel zur Überwachung der Pfropfenbildung ein Mittel (3) zur Überwachung des statischen Druckes im Förderkananl beinhalten und
dass ein abhängiger Regelkreis einen Regler (1.PI) beinhaltet, welcher die messbaren Grössen aufnimmt und verarbeitet und Signale an Mittel (VL) zur Beinflussung des Druckes des Mediums und Mittel (S; Vp) zur Bremsung des Fadens abgibt und dass ein unabhängiger Regelkreis einen Regler (2.PI) beinhaltet, welcher die messbaren Grössen aufnimmt und verarbeitet und Signale an Mittel (HS) zur Beeinflussung der Temperatur des Mediums abgibt.

15. Vorrichtung nach Anspruch 13, **dadurch gekennzeichnet, dass** die Mittel zur Überwachung der Pfropfenbildung ein Mittel (3) zur Messung des Druckes im Förderkanal beinhalten und dass ein abhängiger Regelkreis einen Regler (3.PI, 1.PI) beinhaltet, welcher die gemessenen Drücke aufnimmt und verarbeitet und Signale an Mittel (HS) zur Regelung der Temperatur des Fördermediums abgibt, sowie das je ein unabhänger Regelkreis je einen Regler beinhaltet, wobei der eine Regler (DR) der Regelung der Bremsung des Fadens und der andere (2.PI) der Regelung des Druckes im Fördermedium dient.

16. Vorrichtung nach Anspruch 15, **dadurch gekennzeichnet, dass** der Regler zur Bremsung des Fadens die Anstauluft (S) für das Anstauen des Pfropfens in der Stauchkammer (2) regelt.

17. Vorrichtung nach Anspruch 13, **dadurch gekennzeichnet, dass** die Mittel zur Überwachung der Pfropfenbildung ein Mittel (FSS) zur Überwachung der Fadenspannung nach Auflösung des Pfropfens beinhalten und, dass ein abhängiger Regelkreis einen Regler (3.PI) beinhaltet, welcher die gemessene Fadenspannung verarbeitet und Signale an Mittel (1.PI, VL) zur Regelung eines vorgegebenen Druckes im Fördermedium abgibt und dass je ein unabhängiger Regelkreis je einen Regler beinhaltet, wobei der eine Regler (2.PI) die Temperatur des Mediums und der andere Regler (DR) die Bremsung des Fadens regelt.

18. Vorrichtung nach Anspruch 17, **dadurch gekennzeichnet, dass** es sich bei der Bremsung des Fadens um die Regelung der in die Stauchkammer (2) eingeblasenen Anstauluft (S) handelt, welche den Pfropfen in der Stauchkammer bremst.

19. Vorrichtung nach Anspruch 13, **dadurch gekennzeichnet, dass** die Mittel zur Überwachung der Pfropfenbildung ein optischer Sensor (LS) sind, welcher den Auflösepunkt des Pfropfens an einer Kühltrommel (T) feststellt und dadurch einen Kühlwinkel (β) des sich auf der Kühltrommel befindlichen Pfropfens festlegt und entsprechend ein Signal als messbare Grösse an einen Regler (4.PI) eines abhängigen Regelkreises abgibt, welcher ein weiteres Signal aufgrund des ersten Signales an einen weiteren Regler (1.PI) abgibt, welcher die Bremsung des Fadens regelt und
dass je ein unabhängiger Regelkreis (2.PI; 3.PI) die Temperatur (H) und den Druck (VL) des Fördermediums regeln.

20. Vorrichtung nach Anspruch 19, **dadurch gekennzeichnet, dass** es sich bei der Bremsung des Fadens um eine in die Stauchkammer (2) eingeblasene Anstauluft (S) handelt, welche den Pfropfen in der Stauchkammer bremst.

21. Vorrichtung nach Anspruch 13, **dadurch gekennzeichnet, dass** die Mittel zur Überwachung der Pfropfenbildung ein optischer Sensor (LS) sind, welcher den Auflösepunkt des Pfropfens an einer Kühltrommel (T) feststellt und dadurch einen Kühlwinkel (β) des sich auf der Kühltrommel befindlichen Pfropfens festlegt und entsprechend ein Signal als messbare Grösse an einen Regler (4.PI) eines abhängigen Regelkreises abgibt, welcher ein weiteres Signal an einen weiteren Regler (1.PI) abgibt, welcher die Temperatur (H) des Fördermediums regelt und dass je ein unabhängiger Regelkreis (2.PI; DR) den Druck (VL) des Fördermediums und die Bremsung (S) des Fadens regeln.

22. Vorrichtung nach Anspruch 21, **dadurch gekennzeichnet, dass** es sich bei der Bremsung des Fadens um das Regeln einer in die Stauchkammer (2) eingeblasenen Anstauluft (S) zum Anstauen des Pfropfens in der Stauchkammer handelt.

23. Vorrichtung nach Anspruch 13, **dadurch gekennzeichnet, dass** die Mittel zur Überwachung der Pfropfenbildung ein optischer Sensor (LS) sind, welcher den Auflösepunkt des Pfropfens an einer Kühltrommel (T) feststellt und dadurch einen Kühlwinkel (β) des sich auf der Kühltrommel befindlichen Pfropfens festlegt und entsprechend ein Signal als messbare Grösse an einen Regler (3.PI) eines abhängigen Regelkreises abgibt welcher ein weiteres Signal an einen weiteren Regler (1.PI) abgibt, welcher den Druck (VL) des Fördermediums regelt und dass je ein unabhängiger Regelkreis (2.PI; DR) je die Temperatur (H) des Fördermediums und die Bremsung (S) des Fadens regeln.

24. Vorrichtung nach Anspruch 23, **dadurch gekennzeichnet, dass** es sich bei der Bremsung des Fadens um eine in die Stauchkammer (2) eingeblasene Anstauluft (S) zur Bremsung des Pfropfens in der Stauchkammer handelt.

## Claims

1. Method for continuous crimping of a thread of thermoplastic material with the following process steps:
- compressing a transporting medium;
- heating a transporting medium;
- conveying the thread with a conveying speed through a conveying duct (10) and through an outlet (11) of the conveying duct;
- -conveying the thread from the outlet opening of the conveying duct to the inlet opening of a stuffer box (2);
- -compressing the thread in the stuffer box to a plug by decelerating of the thread;
- conveying the plug from an outlet opening of the stuffer box, with a plug speed which is smaller than the conveying speed, in such a way that the plug is cooled and
- dissolving the plug to a crimped however tensioned thread;
- measuring at least one parameter which is being affected by the plug formation;
- comparing this one measured parameter with a target value for this parameter;
- and transmitting at least one signal to at least one control circuit depending on this parameter, in case of a difference between this measured parameter and the target value,
**characterized in that**,
- the dependent control circuits are of the type of at least one of the following control circuits, i.e.
- a control circuit for the regulation of the afore-mentioned pressure of the transporting medium;
- a control circuit for the regulation of the aforementioned temperature of the transporting medium
- and a control circuit for the regulation of the afore-mentioned deceleration of the thread and;
- that at least one further control circuit, being independent of the aforementioned control circuits, is provided for adjustment of at least;
- the pressure of the transporting medium;
- the temperature of the transporting medium, and
- the deceleration of the thread, if these latter-mentioned parameters are not already being controlled by the dependent control circuits.

2. Method according to claim 1, **characterized in that**, regarding the measured value, said value is the pressure of the transporting medium in the conveying duct (1) and regarding the one dependent control circuit, said circuit is the control circuit for the regulation of the aforementioned pressure of the transporting medium and regarding the other dependent control circuit, said circuit is the control circuit for the regulation of a compression medium (5) blown into the stuffer box (2) for the afore-mentioned deceleration of the thread and regarding the independent control circuit, said circuit is the control circuit for the regulation of the aforementioned temperature of the transporting medium.

3. Method according to claim 1, **characterized in that**, regarding the measured value, said value is a thread tension in the crimped, however, tensioned thread, and regarding the dependent control circuit, said circuit is the control circuit mentioned for the regulation of the pressure mentioned of the transporting medium and that regarding the independent control circuit, one each of said circuit is a control circuit for the regulation of the deceleration of the thread and a control circuit for the regulation of the temperature of the transporting medium.

4. Method according to claim 3, **characterized in that**, regarding the independent control circuit for the deceleration of the thread, said circuit is for the regulation of a stemming air (S) blown into the stuffer box to stem the plug in the stuffer box.

5. Method according to claim 1, **characterized in that**, regarding the measured value, said value is the static pressure in the conveying duct (10) within the zone of the nozzle exit opening (11) and regarding the dependent control circuit, said circuit is for the regulation of the temperature of the transporting medium and that regarding the independent control circuit, one each of said circuits is the control circuit for the regulation of the deceleration of the thread and for the regulation of the pressure for the transporting medium.

6. Method according to claim 5, **characterized in that**, regarding the mentioned independent control circuit for the deceleration of the thread, said circuit is the control circuit for the regulation of a stemming air blown into the stuffer box to stem the plug in the stuffer box.

7. Method according to claim 1, **characterized in that**, regarding the measured value, said value is a cooling angle (β) on a cooling drum (T) to cool the plug and regarding the dependent control circuit, said circuit is the control circuit mentioned for the regulation of the pressure of the transporting medium, in combination with one each of the independent control circuit for the regulation of the deceleration of the thread and for the regulation of the temperature of the transporting medium

8. Method according to claim 7, **characterized in that**, regarding the independent control circuit for the deceleration of the thread, said circuit is the control circuit for the regulation of a stemming air (S) blown into the stuffer box to stem the plug within the stuffer box.

9. Method according to claim 1, **characterized in that**, regarding the measured value, said value is a cooling angle (β) and regarding the dependent control circuit, said control circuit is the control circuit mentioned for the regulation of the deceleration of the thread, in combination with one each of the independent control circuits for the regulation of the pressure and the temperature of the transporting medium.

10. Method according to claim 9, **characterized in that**, regarding the dependent control circuit for the deceleration of the thread, said circuit is the control circuit for the regulation of a stemming air blown into the stuffer box to stem the plug in the stuffer box.

11. Method according to claim 1, **characterized in that**, regarding the measured value, said value is a cooling angle (β) on a cooling drum to cool the plug and regarding the dependent control circuit, said circuit is the control circuit for the regulation of the temperature of the transporting medium and regarding the independent control circuit, said circuit is the control circuit for the regulation of the pressure of the transporting medium and the control circuit for the regulation of the deceleration of the thread.

12. Method according to claim 11, **characterized in that**, regarding the control circuit for the regulation of the deceleration of the thread, said circuit is for the regulation of stemming air (S) blown into the stuffer box to stem the plug in the stuffer box.

13. Device for continuous crimping of a thread made of thermoplastic material, comprising:
- a conveying duct (10) for a thread (F), with an outlet opening (11);
- a source of compressed air (Q) for a transporting medium in connection with the conveying duct;
- a stuffer box (2) with an inlet opening and an outlet opening, in which the thread is formed into a plug;
- coolants to cool the plug;
- means to dissolve the plug to a crimped however tensioned thread;
- means to measure at least one parameter of the plug;
- means to compare at least one parameter with a target value, and
- means to influence the plug formation based on this comparison,
**characterized in that** the means to influence the plug formation comprise at least one of the following, from the result of this comparison, said resulting depending on the control circuit:
- a control circuit for the regulation of a predetermined pressure in the transporting medium;
- a control circuit for the regulation of a predetermined temperature of the transporting medium and;
- a control circuit for the regulation of the deceleration (S; W; VP) of the thread in the stuffer box and at least a further regulating circuit independent of the afore-mentioned control circuits;
- that is, a control circuit for the regulation of a predetermined pressure in the transporting medium;
- a control circuit for the regulation of a predetermined temperature of the transporting medium, and
- a control circuit for the regulation of the deceleration (8; W; Vp) of the thread in the stuffer box.

14. Device according to claim 13, **characterized in that** the means for the monitoring of the plug formation comprise a means (3) for the monitoring of the static pressure in the conveying duct and that a dependent control circuit comprises a regulator (1.PI) which records and processes the measurable values and transmits signals to means (VL) to influence the pressure of the medium and means (S; Vp) for the deceleration of the thread and that an independent control circuit comprises a regulator (2.PI) which records and processes the measurable values and transmits signals to means (HS) to influence the temperature of the medium.

15. Device according to claim 13, **characterized in that** the means for the monitoring of the plug formation comprise a means (3) to measure the pressure in the conveying duct and that a dependent control circuit comprises a regulator (3.PI, 1.PI) which records and processes the measured pressures and transmits signals to means (HS) for the regulation of the temperature of the transporting medium, as well as that one independent control circuit comprises a regulator each, whereby one of the regulators (DR) serves for the regulation of the deceleration of the thread and the other (2.PI) for the regulation of the pressure in the transporting medium.

16. Device according to claim 15, **characterized in that** the regulator for the deceleration of the thread, controls the stemming air (8) to stem the plug in the stuffer box (2).

17. Device according to claim 13, **characterized in that** the means for the monitoring of the plug formation comprise a means (FSS) for the monitoring of the thread tension after the plug has been dissolved and that a dependent control circuit comprises a regulator (3.PI), which processes the measured thread tension and transmits signals to means (1.PI, VL) for the regulation of a predetermined pressure in the transporting medium and that one independent control circuit each comprises a regulator each, whereby one of the regulator (2.PI) controls the temperature of the medium and the other regulator (DR) the deceleration of the thread.

18. Device according to claim 17, **characterized in that**, regarding the deceleration of the thread, said deceleration relates to the regulation of stemming air (S) blown into the stuffer box (2) which decelerates the plug in the stuffer box.

19. Device according to claim 13, **characterized in that** the means to the monitor the plug formation are an optical sensor (LS) which registers the point of dissolution of the plug on a cooling drum (T) and thus determines a cooling angle (β) of the plug located on the cooling drum and accordingly transmits a signal as measurable value to a regulator (4.PI) of a dependent control circuit which transmits a further signal based on the first signal to a further regulator (1.PI) which controls the deceleration of the thread and that an independent control circuit (2.PI; 3.PI) each controls the temperature (H) and the pressure (VL) of the transporting medium.

20. Device according to claim 19, **characterized in that**, regarding the deceleration of the thread, said deceleration of the thread relates to stemming air (s) blown into the stuffer box (2), whereat said air decelerates the plug in the stuffer box.

21. Device according to claim 13, **characterized in that** the means for the monitoring of the plug formation are an optical sensor (LS), which registers the point of dissolution of the plug on a cooling drum (T) and thus determines a cooling angle (β) of the plug located on the cooling drum and accordingly transmits a signal as measurable value to a regulator (4.PI). of a dependent control circuit which transmits a further signal to a further regulator (1.PI) which controls the temperature (H) of the transporting medium and that one independent control circuit each (2.PI; DR) controls the pressure (VL) of the transporting medium and the deceleration (S) of the thread.

22. Device according to claim 21, **characterized in that**, regarding the deceleration of the thread, said deceleration relates to the regulation of a stemming air (S) blown into the stuffer box (2) to stem the plug in the stuffer box.

23. Device according to claim 13, **characterized in that** the means for the monitoring of the plug formation are an optical sensor (LS), which registers the point of dissolution of the plug on a cooling drum (T) and thus determines a cooling angle (β) of the plug located on the cooling drum and accordingly transmits a signal as measurable value to a regulator (3.PI) of a dependent control circuit, which emits a further signal based on the first signal to a further regulator (1.PI) which controls the pressure (VL) of the transporting medium and that an independent control circuit (2.PI; DR) each controls the temperature (H) of the transporting medium and the deceleration (S) of the thread.

24. Device according to claim 23, **characterized in that**, regarding the deceleration of the thread, said deceleration of the thread relates to stemming air (S) blown into the stuffer box (2) for the deceleration of the plug in the stuffer box.

## Revendications

1. Procédé utilisé pour friser continuellement un fil composé de matière thermoplastique, avec les étapes de processus suivantes;
- Compression d'un média de transport
- Echauffement d'un média de transport
- Transport du fil avec une vitesse de transport à travers un canal de transport (10) et à travers une ouverture de sortie (11) du canal de transport
- Transport du fil depuis l'ouverture de sortie du canal de transport dans l'ouverture d'entrée d'une chambre de refoulement (2)
- Compression du fil dans la chambre de refoulement en un bouchon par décélération du fil
- Transport du bouchon depuis une ouverture de sortie de la chambre de refoulement, avec une vitesse de bouchon qui est plus petite que la vitesse de transport, de sorte que le bouchon est refroidi, et
- Désagrégation du bouchon en un fil frisé, cependant tendu
- Mesurer au moins un paramètre influencé par la formation du bouchon
- Comparer ce paramètre mesuré avec une valeur de consigne pour ce paramètre
- Et transmettre au moins un signal à au moins un circuit de réglage dépendant de ce paramètre, dans le cas d'une différence entre ce paramètre mesuré et la valeur de consigne
- **caractérisé par le fait que,**
- en ce qui concerne les circuits de réglage dépendants, il s'agit d'au moins un des circuits de réglage suivants, c'est-à-dire
- un circuit de réglage utilisé pour moduler la pression citée précédemment du média de transport,
- un circuit de réglage utilisé pour moduler la température citée précédemment du média de transport,
- et un circuit de réglage utilisé pour moduler le freinage cité précédemment du fil, et
- qu'au moins un autre circuit de réglage, indépendant des circuits de réglage cités précédemment, est prévu, et ceci pour la modulation d'au moins
- la pression du média de transport
- la température du média de transport, et
- le freinage du fil,
lorsque ces paramètres cités en derniers ne sont pas déjà modulés par les circuits de réglage dépendants.

2. Procédé selon revendication 1,
**caractérisé par le fait que**,
en ce qui concerne la valeur mesurée, il s'agit de la pression du média de transport dans le canal de transport (1), et, pour un des circuits de réglage dépendants, il s'agit du circuit de réglage utilisé pour la modulation de la pression citée précédemment du média de transport, et pour l'autre circuit de réglage dépendant, il s'agit du circuit de réglage utilisé pour la modulation d'un média de refoulement (5) soufflé dans la chambre de refoulement (2), pour le freinage du fil cité précédemment, et pour le circuit de réglage indépendant, il s'agit du circuit de réglage utilisé pour la modulation de la température citée précédemment du média de transport.

3. Procédé selon revendication 1,
**caractérisé par le fait que,**
en ce qui concerne la valeur mesurée, il s'agit d'une tension de fil d'un fil frisé, cependant tendu, et, en ce qui concerne le circuit de réglage dépendant, il s'agit dudit circuit de réglage utilisé pour la modulation de ladite pression du média de transport, et que, en ce qui concerne le circuit de réglage indépendant, il s'agit, à chaque fois, d'un circuit de réglage utilisé pour la modulation du freinage du fil, et d'un circuit de réglage utilisé pour la modulation de la température du média de transport.

4. Procédé selon revendication 3,
**caractérisé par le fait que,**
en ce qui concerne le circuit de réglage indépendant servant au freinage du fil, il s'agit de la modulation d'un air de refoulement (S) soufflé dans la chambre de refoulement, afin de refouler le bouchon dans la chambre de refoulement.

5. Procédé selon revendication 1,
**caractérisé par le fait que**,
en ce qui concerne la valeur mesurée, il s'agit de la pression statique dans le canal de transport (10), dans la zone de l'ouverture de sortie de buse (11), et, en ce qui concerne le circuit de réglage dépendant, il s'agit de la modulation de la température du média de transport, et que, en ce qui concerne le circuit de réglage indépendant, il s'agit, à chaque fois, d'un circuit de réglage utilisé pour la modulation du freinage du fil, et d'un circuit de réglage utilisé pour la modulation de la pression du média de transport.

6. Procédé selon revendication 5,
**caractérisé par le fait que**,
en ce qui concerne ledit circuit de réglage indépendant servant au freinage du fil, il s'agit du circuit de réglage utilisé pour la modulation d'un air de refoulement soufflé dans la chambre de refoulement, afin de refouler le bouchon dans la chambre de refoulement.

7. Procédé selon revendication 1,
**caractérisé par le fait que**,
en ce qui concerne la valeur mesurée, il s'agit d'un angle de refroidissement (β) sur un tambour de refroidissement (T) utilisé pour le refroidissement du bouchon, et, en ce qui concerne le circuit de réglage dépendant, il s'agit dudit circuit de réglage utilisé pour la modulation de la pression du média de transport, en combinaison, à chaque fois, avec un circuit de réglage indépendant utilisé pour la modulation du freinage du fil, et pour la modulation de la température du média de transport.

8. Procédé selon revendication 7,
**caractérisé par le fait que**,
en ce qui concerne le circuit de réglage indépendant utilisé pour le freinage du fil, il s'agit du circuit de réglage utilisé pour la modulation d'un air de refoulement (S) soufflé dans la chambre de refoulement, afin de refouler le bouchon dans la chambre de refoulement.

9. Procédé selon revendication 1,
**caractérisé par le fait que**,
en ce qui concerne la'valeur mesurée, il s'agit d'un angle de refroidissement (β), et, en ce qui concerne le circuit de réglage dépendant, il s'agit dudit circuit de réglage utilisé pour la modulation du freinage du fil, en combinaison, à chaque fois, avec un circuit de réglage indépendant utilisé pour la modulation de la pression, et de la température du média de transport.

10. Procédé selon revendication 9,
**caractérisé par le fait que**,
en ce qui concerne le circuit de réglage dépendant utilisé pour le freinage du fil, il s'agit du circuit de réglage utilisé pour la modulation d'un air de refoulement soufflé dans la chambre de refoulement, afin de refouler le bouchon dans la chambre de refoulement.

11. Procédé selon revendication 1,
**caractérisé par le fait que,**
en ce qui concerne la valeur mesurée, il s'agit d'un angle de refroidissement (β) sur un tambour de refroidissement utilisé pour le refroidissement du bouchon, et, en ce qui concerne le circuit de réglage dépendant, il s'agit du circuit de réglage utilisé pour la modulation de la température du média de transport, et, en ce qui concerne le circuit de réglage indépendant, il s'agit du circuit de réglage utilisé pour la modulation de la pression du média de transport et du circuit de réglage utilisé pour la modulation du freinage du fil.

12. Procédé selon revendication 11,
**caractérisé par le fait que**,
en ce qui concerne le circuit de réglage utilisé pour la modulation du freinage du fil, il s'agit de la modulation d'un air de refoulement (S) soufflé dans la chambre de refoulement, afin de refouler le bouchon dans la chambre de refoulement.

13. Dispositif utilisé pour friser continuellement un fil composé de matière thermoplastique, comprenant:
- un canal de transport (10) pour un fil (F), avec une ouverture de sortie (11) ;
- une source d'air comprimé (Q) pour un média de transport en liaison avec le canal de transport;
- une chambre de refoulement (2) avec une ouverture d'entrée et une ouverture de sortie, dans laquelle le fil est formé en un bouchon;
- moyens réfrigérants pour le refroidissement du bouchon;
- moyens pour la désagrégation du bouchon en un fil frisé, cependant tendu;
- moyens pour la mesure d'au moins un paramètre du bouchon;
- moyens pour comparer au moins ce seul paramètre avec une valeur de consigne; et
- moyens pour influencer la formation du bouchon en vertu de cette comparaison,
**caractérisé par le fait que**
les moyens pour influencer la formation du bouchon comprennent au moins un des circuits de réglage suivants, dépendants du résultat de cette comparaison:
- un circuit de réglage pour la modulation d'une pression prédéterminée du média de transport
- un circuit de réglage pour la modulation d'une température prédéterminée du média de transport, et
- un circuit de réglage pour la modulation du freinage (S ; W ; Vp) du fil dans la chambre de refoulement
et au moins un autre circuit de réglage, indépendant des circuits de réglage cités précédemment
- c'est-à-dire un circuit de réglage pour la modulation d'une pression prédéterminée du média de transport
- un circuit de réglage pour la modulation d'une température prédéterminée du média de transport, et
- un circuit de réglage pour la modulation du freinage (S ; W ; Vp) du fil dans la chambre de refoulement.

14. Dispositif selon revendication 13,
**caractérisé par le fait que**
les moyens utilisés pour la surveillance de la formation du bouchon comprennent un moyen (3) pour la surveillance de la pression statique dans le canal de transport, et qu'un circuit de réglage dépendant comprend un régleur (1.PI) qui réceptionne et travaille les valeurs mesurables, et émet des signaux vers des moyens (VL) pour influencer la pression du média et vers des moyens (S ; Vp) pour le freinage du fil, et qu'un circuit de réglage indépendant comprend un régleur (2.PI) qui réceptionne et travaille les valeurs mesurables, et émet des signaux vers des moyens (HS) pour influencer la température du média.

15. Dispositif selon revendication 13,
**caractérisé par le fait que**
les moyens utilisés pour la surveillance de la formation du bouchon comprennent un moyen (3) pour la mesure de la pression dans le canal de transport, et qu'un circuit de réglage dépendant comprend un régleur (3.PI,1.PI) qui réceptionne et travaille les pressions mesurées, et émet des signaux vers des moyens (HS) pour la modulation de la température du média de transport, de même que chacun des circuits de réglage indépendants comprend un régleur, et où l'un des régleurs (DR) sert à la modulation du freinage du fil, et l'autre (2.PI) sert à la modulation de la pression du média de transport.

16. Dispositif selon revendication 15,
**caractérisé par le fait que**
le régleur utilisé pour le freinage du fil règle l'air de refoulement (S) pour le refoulement du bouchon dans la chambre de refoulement (2).

17. Dispositif selon revendication 13,
**caractérisé par le fait que**
les moyens utilisés pour la surveillance de la formation du bouchon comprennent un moyen (FSS) pour la surveillance de la tension du fil, après désagrégation du bouchon, et
qu'un circuit de réglage dépendant comprend un régleur (3.PI) qui travaille la tension de fil mesurée et émet des signaux vers des moyens (1.PI,VL) pour la modulation d'une pression prédéterminée du média de transport, et que chacun des circuits de réglage indépendants comprend un régleur, et où l'un des régleurs (2.PI) règle la température du média, et l'autre régleur (DR) règle le freinage du fil.

18. Dispositif selon revendication 17,
**caractérisé par le fait que**,
en ce qui concerne le freinage du fil, il s'agit de la modulation de l'air de refoulement (S) soufflé dans la chambre de refoulement (2) qui freine le bouchon dans la chambre de refoulement.

19. Dispositif selon revendication 13,
**caractérisé par le fait que**
les moyens utilisés pour la surveillance de la formation du bouchon sont constitués par un détecteur optique (LS) qui constate le point de désagrégation du bouchon sur un tambour de refroidissement (T), et détermine, par cela, un angle de refroidissement (β) du bouchon se trouvant sur le tambour de refroidissement, et
émet, en correspondance, un signal comme valeur mesurable vers un régleur (4.PI) d'un circuit de réglage dépendant, lequel émet un autre signal, en vertu du premier signal, vers un autre régleur (1.PI) qui règle le freinage du fil, et
que chacun des circuits de réglage indépendants (2.PI ;3.PI) règle la température (H), et la pression (VL) du média de transport.

20. Dispositif selon revendication 19,
**caractérisé par le fait que,**
en ce qui concerne le freinage du fil, il s'agit d'un air de refoulement (S) soufflé dans la chambre de refoulement (2) qui freine le bouchon dans la chambre de refoulement.

21. Dispositif selon revendication 13,
**caractérisé par le fait que**
les moyens utilisés pour la surveillance de la formation du bouchon sont constitués par un détecteur optique (LS) qui constate le point de désagrégation du bouchon sur un tambour de refroidissement (T), et détermine, par cela, un angle de refroidissement (β) du bouchon se trouvant sur le tambour de refroidissement, et émet, en correspondance, un signal comme valeur mesurable vers un régleur (4.PI) d'un circuit de réglage dépendant, lequel émet un autre signal vers un autre régleur (1.PI) qui règle la température (H) du média de transport, et
que chacun des circuits de réglage indépendants (2.PI ;DR) règle la pression (VL) du média de transport, et le freinage (S) du fil.

22. Dispositif selon revendication 21,
**caractérisé par le fait que**,
en ce qui concerne le freinage du fil, il s'agit de la modulation d'un air de refoulement (S) soufflé dans la chambre de refoulement (2) pour refouler le bouchon dans la chambre de refoulement.

23. Dispositif selon revendication 13,
**caractérisé par le fait que**
les moyens utilisés pour la surveillance de la formation du bouchon sont constitués par un détecteur optique (LS) qui constate le point de désagrégation du bouchon sur un tambour de refroidissement (T), et détermine, par cela, un angle de refroidissement (β) du bouchon se trouvant sur le tambour de refroidissement, et émet, en correspondance, un signal comme valeur
mesurable vers un régleur (3.PI) d'un circuit de réglage dépendant, lequel émet un autre signal vers un autre régleur (1.PI) qui règle la pression (VL) du média de transport, et
que chacun des circuits de réglage indépendants (2.PI ;DR) règle la température (H) du média de transport, et le freinage (S) du fil.

24. Dispositif selon revendication 23,
**caractérisé par le fait que**,
en ce qui concerne le freinage du fil, il s'agit d'un air de refoulement (S) soufflé dans la chambre de refoulement (2) qui freine le bouchon dans la chambre de refoulement.
